# EUROPEAN PATENT APPLICATION

(11) **EP 3 825 404 A1**
(43) Date of publication of application: **26.05.2021**
(21) Application number: 19838454.7
(22) Date of filing: 16.07.2019
(51) Int. Cl.: C12N 15/13, A61K 35/17, A61P 35/00, C07K 16/28, C07K 16/46, C07K 19/00, C12N 5/10, C12N 15/63, C07K 7/06, C07K 7/08

(54) **ANTI-GPC3 SINGLE-CHAIN ANTIBODY-CONTAINING CAR**

(30) Priority: 17.07.2018 JP 2018133970
(71) Applicant: Noile-Immune Biotech, Inc., Tokyo 1050012 (JP)
(72) Inventor: TAMADA, Koji, Ube-shi, Yamaguchi 755-8505 (JP); SAKODA, Yukimi, Ube-shi, Yamaguchi 755-8505 (JP); ADACHI, Keishi, Ube-shi, Yamaguchi 755-8505 (JP)
(74) Representative: Dennemeyer & Associates S.A.
(86) International application number: PCT/JP2019/027835
(87) International publication number: WO 2020/017479

(57) **Abstract**

The present invention aims to provide a CAR that contains a single-chain antibody capable of specifically binding to GPC3 localized in the cell membrane and is useful for cancer immunotherapy using a CAR-T cell; and an immunocompetent cell expressing such a CAR. A gene encoding a CAR containing: a single-chain antibody that contains specific heavy-chain CDR1 to CDR3 and specific light-chain CDR1 to CDR3, as defined in claim 1, and specifically binds to a human-derived GPC3 polypeptide; a transmembrane region fused to a carboxyl terminus of the single-chain antibody; and an immunocompetent cell activation signaling transduction region fused to a carboxyl terminus of the transmembrane region is produced, and the gene is introduced into an immunocompetent cell. Thereby, a CAR-expressing immunocompetent cell having excellent cytotoxic activity on cancer cells and interferon gamma (IFN-γ) producibility can be produced.

SEQUENCE LISTING

<110>  Noile-Immune Biotech, Inc.

<120>  CAR comprising anti-GPC3 scFv

<130>  KT-08P

<150>  2018-133970
<151>  2018-07-17

<160>  19

<170>  PatentIn version 3.1

<210>  1
<211>  5
<212>  PRT
<213>  Artificial

<220>
<223>  H chain CDR1 of GC33 antibody

<220>
<221>  MISC_FEATURE
<223>  Inventor: Tamada, Koji; Sakoda, Yukimi; Adachi, Keishi


<400>  1

Asp Tyr Glu Met His
1               5


<210>  2
<211>  17
<212>  PRT
<213>  Artificial

<220>
<223>  H chain CDR2 of GC33 antibody

<400>  2

Ala Leu Asp Pro Lys Thr Gly Asp Thr Ala Tyr Ser Gln Lys Phe Lys
1               5                   10                  15


Gly




<210>  3
<211>  6
<212>  PRT
<213>  Artificial

<220>
<223>  H chain CDR3 of GC33 antibody

<400>  3

Phe Tyr Ser Tyr Thr Tyr

1               5


<210>  4
<211>  16
<212>  PRT
<213>  Artificial

<220>
<223>  L chain CDR1 of GC33 antibody

<400>  4

Arg Ser Ser Gln Ser Leu Val His Ser Asn Gly Asn Thr Tyr Leu His
1               5                   10                  15


<210>  5
<211>  7
<212>  PRT
<213>  Artificial

<220>
<223>  L chain CDR2 of GC33 antibody

<400>  5

Lys Val Ser Asn Arg Phe Ser
1               5


<210>  6
<211>  9
<212>  PRT
<213>  Artificial

<220>
<223>  L chain CDR3 of GC33 antibody

<400>  6

Ser Gln Asn Thr His Val Pro Pro Thr
1               5


<210>  7
<211>  114
<212>  PRT
<213>  Artificial

<220>
<223>  H chain V region of GC33 antibody

<400>  7

Gln Val Gln Leu Gln Gln Ser Gly Ala Glu Leu Val Arg Pro Gly Ala
1               5                   10                  15


Ser Val Lys Leu Ser Cys Lys Ala Ser Gly Tyr Thr Phe Thr Asp Tyr
            20                  25                  30

Glu Met His Trp Val Lys Gln Thr Pro Val His Gly Leu Lys Trp Ile
        35                  40                  45

Gly Ala Leu Asp Pro Lys Thr Gly Asp Thr Ala Tyr Ser Gln Lys Phe
    50                  55                  60

Lys Gly Lys Ala Thr Leu Thr Ala Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser

<210>  8
<211>  113
<212>  PRT
<213>  Artificial

<220>
<223>  H chain L region of GC33 antibody

<400>  8

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Asn
                85                  90                  95

Thr His Val Pro Pro Thr Phe Gly Ser Gly Thr Lys Leu Glu Ile Lys
            100                 105                 110

Arg

<210>  9
<211>  342
<212>  DNA
<213>  Artificial

<220>
<223>  H chain V region gene of GC33 antibody

<400>  9
caggtgcagc tgcagcagag cggagctgag ctggtgagac ctggcgccag cgtgaaactg      60

agctgcaagg ccagcggcta caccttcacc gactacgaga tgcactgggt gaagcagaca     120

cccgtgcacg gcctgaagtg gatcggagcc ctggacccca agaccggaga caccgcctac     180

agccagaagt ttaagggcaa ggccaccctc accgccgata agagctccag caccgcctac     240

atggagctga ggagcctgac ctccgaggac agcgccgtgt actattgcac caggttctac     300

agctacacct actggggcca gggaacactg gtgaccgtct cc                        342


<210>  10
<211>  339
<212>  DNA
<213>  Artificial

<220>
<223>  H chain L region gene of GC33 antibody

<400>  10
gatgtggtga tgacacagac ccctctgagc ctgcctgtga gcctgggaga ccaggccagc      60

atcagctgca ggagcagcca gagcctggtg cacagcaacg gcaacacata cctccactgg     120

tacctgcaga agcctggcca gagccccaag ctgctgatct acaaggtgag caacaggttc     180

tccggcgtgc ccgatagatt cagcggctcc ggcagcggca ccgactttac cctgaagatc     240

tccagagtgg aggccgagga cctgggcgtg tacttttgca gccagaacac ccacgtgcct     300

cctaccttcg gctccggcac caagctcgaa atcaagagg                            339


<210>  11
<211>  27
<212>  PRT
<213>  Homo sapiens

<220>
<221>  MISC_FEATURE
<223>  537 th glycine to 563 th of human GPC3


<400>  11

Gly Asn Ser Gln Gln Ala Thr Pro Lys Asp Asn Glu Ile Ser Thr Phe
1               5                   10                  15

His Asn Leu Gly Asn Val His Ser Pro Leu Lys
            20                  25

<210>  12
<211>  242
<212>  PRT
<213>  Artificial

<220>
<223>  humanized scFv of GC33 antibody

<400>  12

Gln Val Gln Leu Gln Gln Ser Gly Ala Glu Leu Val Arg Pro Gly Ala
1               5                   10                  15

Ser Val Lys Leu Ser Cys Lys Ala Ser Gly Tyr Thr Phe Thr Asp Tyr
            20                  25                  30

Glu Met His Trp Val Lys Gln Thr Pro Val His Gly Leu Lys Trp Ile
        35                  40                  45

Gly Ala Leu Asp Pro Lys Thr Gly Asp Thr Ala Tyr Ser Gln Lys Phe
    50                  55                  60

Lys Gly Lys Ala Thr Leu Thr Ala Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Gly Gly Gly Gly Ser Gly Gly Gly Gly Ser Gly Gly Gly Gly
        115                 120                 125

Ser Asp Val Val Met Thr Gln Thr Pro Leu Ser Leu Pro Val Ser Leu
    130                 135                 140

Gly Asp Gln Ala Ser Ile Ser Cys Arg Ser Ser Gln Ser Leu Val His
145                 150                 155                 160

Ser Asn Gly Asn Thr Tyr Leu His Trp Tyr Leu Gln Lys Pro Gly Gln
                165                 170                 175

Ser Pro Lys Leu Leu Ile Tyr Lys Val Ser Asn Arg Phe Ser Gly Val
            180                 185                 190

Pro Asp Arg Phe Ser Gly Ser Gly Ser Gly Thr Asp Phe Thr Leu Lys

195                 200                 205

Ile Ser Arg Val Glu Ala Glu Asp Leu Gly Val Tyr Phe Cys Ser Gln
    210                 215                 220

Asn Thr His Val Pro Pro Thr Phe Gly Ser Gly Thr Lys Leu Glu Ile
225                 230                 235                 240

Lys Arg

<210>  13
<211>  242
<212>  PRT
<213>  Artificial

<220>
<223>  humanized scFv Ver.2 of GC33 antibody

<400>  13

Asp Val Val Met Thr Gln Thr Pro Leu Ser Leu Pro Val Ser Leu Gly
1               5                   10                  15

Asp Gln Ala Ser Ile Ser Cys Arg Ser Ser Gln Ser Leu Val His Ser
            20                  25                  30

Asn Gly Asn Thr Tyr Leu His Trp Tyr Leu Gln Lys Pro Gly Gln Ser
        35                  40                  45

Pro Lys Leu Leu Ile Tyr Lys Val Ser Asn Arg Phe Ser Gly Val Pro
    50                  55                  60

Asp Arg Phe Ser Gly Ser Gly Ser Gly Thr Asp Phe Thr Leu Lys Ile
65                  70                  75                  80

Ser Arg Val Glu Ala Glu Asp Leu Gly Val Tyr Phe Cys Ser Gln Asn
                85                  90                  95

Thr His Val Pro Pro Thr Phe Gly Ser Gly Thr Lys Leu Glu Ile Lys
            100                 105                 110

Arg Gly Gly Gly Gly Ser Gly Gly Gly Gly Ser Gly Gly Gly Gly Ser
        115                 120                 125

Gln Val Gln Leu Gln Gln Ser Gly Ala Glu Leu Val Arg Pro Gly Ala
    130                 135                 140

Ser Val Lys Leu Ser Cys Lys Ala Ser Gly Tyr Thr Phe Thr Asp Tyr
145                 150                 155                 160

Glu Met His Trp Val Lys Gln Thr Pro Val His Gly Leu Lys Trp Ile
                    165                 170                 175

Gly Ala Leu Asp Pro Lys Thr Gly Asp Thr Ala Tyr Ser Gln Lys Phe
                180                 185                 190

Lys Gly Lys Ala Thr Leu Thr Ala Asp Lys Ser Ser Ser Thr Ala Tyr
        195                 200                 205

Met Glu Leu Arg Ser Leu Thr Ser Glu Asp Ser Ala Val Tyr Tyr Cys
    210                 215                 220

Thr Arg Phe Tyr Ser Tyr Thr Tyr Trp Gly Gln Gly Thr Leu Val Thr
225                 230                 235                 240

Val Ser

<210>  14
<211>  283
<212>  PRT
<213>  Artificial

<220>
<223>  hCD8-hCD28-h4-1BB-hCD3 peptide 1

<400>  14

Phe Val Pro Val Phe Leu Pro Ala Lys Pro Thr Thr Thr Pro Ala Pro
1               5                   10                  15

Arg Pro Pro Thr Pro Ala Pro Thr Ile Ala Ser Gln Pro Leu Ser Leu
            20                  25                  30

Arg Pro Glu Ala Cys Arg Pro Ala Ala Gly Gly Ala Val His Thr Arg
        35                  40                  45

Gly Leu Asp Phe Ala Cys Asp Ile Tyr Ile Trp Ala Pro Leu Ala Gly
    50                  55                  60

Thr Cys Gly Val Leu Leu Leu Ser Leu Val Ile Thr Leu Tyr Cys Asn
65                  70                  75                  80

His Arg Asn Arg Ser Lys Arg Ser Arg Leu Leu His Ser Asp Tyr Met
                85                  90                  95

Asn Met Thr Pro Arg Arg Pro Gly Pro Thr Arg Lys His Tyr Gln Pro
            100                 105                 110

Tyr Ala Pro Pro Arg Asp Phe Ala Ala Tyr Arg Ser Arg Phe Ser Val
        115                 120                 125

Val Lys Arg Gly Arg Lys Lys Leu Leu Tyr Ile Phe Lys Gln Pro Phe
    130                 135                 140

Met Arg Pro Val Gln Thr Thr Gln Glu Glu Asp Gly Cys Ser Cys Arg
145                 150                 155                 160

Phe Pro Glu Glu Glu Glu Gly Gly Cys Glu Leu Arg Val Lys Phe Ser
                165                 170                 175

Arg Ser Ala Asp Ala Pro Ala Tyr Gln Gln Gly Gln Asn Gln Leu Tyr
            180                 185                 190

Asn Glu Leu Asn Leu Gly Arg Arg Glu Glu Tyr Asp Val Leu Asp Lys
        195                 200                 205

Arg Arg Gly Arg Asp Pro Glu Met Gly Gly Lys Pro Arg Arg Lys Asn
    210                 215                 220

Pro Gln Glu Gly Leu Tyr Asn Glu Leu Gln Lys Asp Lys Met Ala Glu
225                 230                 235                 240

Ala Tyr Ser Glu Ile Gly Met Lys Gly Glu Arg Arg Arg Gly Lys Gly
                245                 250                 255

His Asp Gly Leu Tyr Gln Gly Leu Ser Thr Ala Thr Lys Asp Thr Tyr
            260                 265                 270

Asp Ala Leu His Met Gln Ala Leu Pro Pro Arg
        275                 280

<210>  15
<211>  277
<212>  PRT
<213>  Artificial

<220>
<223>  hCD8-hCD28-h4-1BB-hCD3 peptide 2

<400>  15

Phe Val Pro Val Phe Leu Pro Ala Lys Pro Thr Thr Thr Pro Ala Pro
1               5                   10                  15

Arg Pro Pro Thr Pro Ala Pro Thr Ile Ala Ser Gln Pro Leu Ser Leu
            20                  25                  30

Arg Pro Glu Ala Cys Arg Pro Ala Ala Gly Gly Ala Val His Thr Arg
        35                  40                  45

Gly Leu Asp Phe Ala Cys Asp Ile Tyr Ile Trp Ala Pro Leu Ala Gly
    50                  55                  60

Thr Cys Gly Val Leu Leu Leu Ser Leu Val Ile Thr Leu Arg Ser Lys
65                  70                  75                  80

Arg Ser Arg Leu Leu His Ser Asp Tyr Met Asn Met Thr Pro Arg Arg
                85                  90                  95

Pro Gly Pro Thr Arg Lys His Tyr Gln Pro Tyr Ala Pro Pro Arg Asp
            100                 105                 110

Phe Ala Ala Tyr Arg Ser Arg Phe Ser Val Val Lys Arg Gly Arg Lys
        115                 120                 125

Lys Leu Leu Tyr Ile Phe Lys Gln Pro Phe Met Arg Pro Val Gln Thr
    130                 135                 140

Thr Gln Glu Glu Asp Gly Cys Ser Cys Arg Phe Pro Glu Glu Glu Glu
145                 150                 155                 160

Gly Gly Cys Glu Leu Arg Val Lys Phe Ser Arg Ser Ala Asp Ala Pro
                165                 170                 175

Ala Tyr Gln Gln Gly Gln Asn Gln Leu Tyr Asn Glu Leu Asn Leu Gly
            180                 185                 190

Arg Arg Glu Glu Tyr Asp Val Leu Asp Lys Arg Arg Gly Arg Asp Pro
        195                 200                 205

Glu Met Gly Gly Lys Pro Arg Arg Lys Asn Pro Gln Glu Gly Leu Tyr
    210                 215                 220

Asn Glu Leu Gln Lys Asp Lys Met Ala Glu Ala Tyr Ser Glu Ile Gly
225                 230                 235                 240

Met Lys Gly Glu Arg Arg Arg Gly Lys Gly His Asp Gly Leu Tyr Gln
                245                 250                 255

Gly Leu Ser Thr Ala Thr Lys Asp Thr Tyr Asp Ala Leu His Met Gln
            260                 265                 270

Ala Leu Pro Pro Arg
        275

<210>  16
<211>  276
<212>  PRT
<213>  Artificial

<220>
<223>  hCD8-hCD28-h4-1BB-hCD3 peptide 3

<400>  16

Phe Val Pro Val Phe Leu Pro Ala Lys Pro Thr Thr Thr Pro Ala Pro
1               5                   10                  15

Arg Pro Pro Thr Pro Ala Pro Thr Ile Ala Ser Gln Pro Leu Ser Leu
            20                  25                  30

Arg Pro Glu Ala Cys Arg Pro Ala Ala Gly Gly Ala Val His Thr Arg
        35                  40                  45

Gly Leu Asp Phe Ala Cys Asp Ile Tyr Ile Trp Ala Pro Leu Ala Gly
    50                  55                  60

Thr Cys Gly Val Leu Leu Leu Ser Leu Val Ile Thr Arg Ser Lys Arg
65                  70                  75                  80

Ser Arg Leu Leu His Ser Asp Tyr Met Asn Met Thr Pro Arg Arg Pro
                85                  90                  95

Gly Pro Thr Arg Lys His Tyr Gln Pro Tyr Ala Pro Pro Arg Asp Phe
            100                 105                 110

Ala Ala Tyr Arg Ser Arg Phe Ser Val Val Lys Arg Gly Arg Lys Lys
        115                 120                 125

Leu Leu Tyr Ile Phe Lys Gln Pro Phe Met Arg Pro Val Gln Thr Thr
    130                 135                 140

Gln Glu Glu Asp Gly Cys Ser Cys Arg Phe Pro Glu Glu Glu Glu Gly
145                 150                 155                 160

Gly Cys Glu Leu Arg Val Lys Phe Ser Arg Ser Ala Asp Ala Pro Ala
                165                 170                 175

Tyr Gln Gln Gly Gln Asn Gln Leu Tyr Asn Glu Leu Asn Leu Gly Arg
            180                 185                 190

Arg Glu Glu Tyr Asp Val Leu Asp Lys Arg Arg Gly Arg Asp Pro Glu
        195                 200                 205

Met Gly Gly Lys Pro Arg Arg Lys Asn Pro Gln Glu Gly Leu Tyr Asn
210                 215                 220

Glu Leu Gln Lys Asp Lys Met Ala Glu Ala Tyr Ser Glu Ile Gly Met
225                 230                 235                 240

Lys Gly Glu Arg Arg Arg Gly Lys Gly His Asp Gly Leu Tyr Gln Gly
                245                 250                 255

Leu Ser Thr Ala Thr Lys Asp Thr Tyr Asp Ala Leu His Met Gln Ala
            260                 265                 270

Leu Pro Pro Arg
        275

<210>  17
<211>  525
<212>  PRT
<213>  Artificial

<220>
<223>  CAR comprising humanized scFv of GC33 antibody

<400>  17

Gln Val Gln Leu Gln Gln Ser Gly Ala Glu Leu Val Arg Pro Gly Ala
1               5                   10                  15

Ser Val Lys Leu Ser Cys Lys Ala Ser Gly Tyr Thr Phe Thr Asp Tyr
            20                  25                  30

Glu Met His Trp Val Lys Gln Thr Pro Val His Gly Leu Lys Trp Ile
        35                  40                  45

Gly Ala Leu Asp Pro Lys Thr Gly Asp Thr Ala Tyr Ser Gln Lys Phe
    50                  55                  60

Lys Gly Lys Ala Thr Leu Thr Ala Asp Lys Ser Ser Ser Thr Ala Tyr
65                  70                  75                  80

Met Glu Leu Arg Ser Leu Thr Ser Glu Asp Ser Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Gly Gly Gly Gly Ser Gly Gly Gly Gly Ser Gly Gly Gly Gly
        115                 120                 125

Ser Asp Val Val Met Thr Gln Thr Pro Leu Ser Leu Pro Val Ser Leu

130                 135                 140

Gly Asp Gln Ala Ser Ile Ser Cys Arg Ser Ser Gln Ser Leu Val His
145                 150                 155                 160

Ser Asn Gly Asn Thr Tyr Leu His Trp Tyr Leu Gln Lys Pro Gly Gln
                165                 170                 175

Ser Pro Lys Leu Leu Ile Tyr Lys Val Ser Asn Arg Phe Ser Gly Val
            180                 185                 190

Pro Asp Arg Phe Ser Gly Ser Gly Ser Gly Thr Asp Phe Thr Leu Lys
        195                 200                 205

Ile Ser Arg Val Glu Ala Glu Asp Leu Gly Val Tyr Phe Cys Ser Gln
    210                 215                 220

Asn Thr His Val Pro Pro Thr Phe Gly Ser Gly Thr Lys Leu Glu Ile
225                 230                 235                 240

Lys Arg Phe Val Pro Val Phe Leu Pro Ala Lys Pro Thr Thr Thr Pro
                245                 250                 255

Ala Pro Arg Pro Pro Thr Pro Ala Pro Thr Ile Ala Ser Gln Pro Leu
            260                 265                 270

Ser Leu Arg Pro Glu Ala Cys Arg Pro Ala Ala Gly Gly Ala Val His
        275                 280                 285

Thr Arg Gly Leu Asp Phe Ala Cys Asp Ile Tyr Ile Trp Ala Pro Leu
    290                 295                 300

Ala Gly Thr Cys Gly Val Leu Leu Leu Ser Leu Val Ile Thr Leu Tyr
305                 310                 315                 320

Cys Asn His Arg Asn Arg Ser Lys Arg Ser Arg Leu Leu His Ser Asp
                325                 330                 335

Tyr Met Asn Met Thr Pro Arg Arg Pro Gly Pro Thr Arg Lys His Tyr
            340                 345                 350

Gln Pro Tyr Ala Pro Pro Arg Asp Phe Ala Ala Tyr Arg Ser Arg Phe
        355                 360                 365

Ser Val Val Lys Arg Gly Arg Lys Lys Leu Leu Tyr Ile Phe Lys Gln
    370                 375                 380

Pro Phe Met Arg Pro Val Gln Thr Thr Gln Glu Glu Asp Gly Cys Ser
385                 390                 395                 400

Cys Arg Phe Pro Glu Glu Glu Glu Gly Gly Cys Glu Leu Arg Val Lys
                405                 410                 415

Phe Ser Arg Ser Ala Asp Ala Pro Ala Tyr Gln Gln Gly Gln Asn Gln
            420                 425                 430

Leu Tyr Asn Glu Leu Asn Leu Gly Arg Arg Glu Glu Tyr Asp Val Leu
        435                 440                 445

Asp Lys Arg Arg Gly Arg Asp Pro Glu Met Gly Gly Lys Pro Arg Arg
    450                 455                 460

Lys Asn Pro Gln Glu Gly Leu Tyr Asn Glu Leu Gln Lys Asp Lys Met
465                 470                 475                 480

Ala Glu Ala Tyr Ser Glu Ile Gly Met Lys Gly Glu Arg Arg Arg Gly
                485                 490                 495

Lys Gly His Asp Gly Leu Tyr Gln Gly Leu Ser Thr Ala Thr Lys Asp
            500                 505                 510

Thr Tyr Asp Ala Leu His Met Gln Ala Leu Pro Pro Arg
        515                 520                 525

<210>  18
<211>  1277
<212>  PRT
<213>  Artificial

<220>
<223>  anti GPC3 scFv CAR+hIL-7+hCCL19+HSV-TK

<400>  18

Met Asp Trp Thr Trp Arg Ile Leu Phe Leu Val Ala Ala Ala Thr Gly
1               5                   10                  15

Ala His Ser Ser Gln Val Gln Leu Gln Gln Ser Gly Ala Glu Leu Val
            20                  25                  30

Arg Pro Gly Ala Ser Val Lys Leu Ser Cys Lys Ala Ser Gly Tyr Thr
        35                  40                  45

Phe Thr Asp Tyr Glu Met His Trp Val Lys Gln Thr Pro Val His Gly
    50                  55                  60

Leu Lys Trp Ile Gly Ala Leu Asp Pro Lys Thr Gly Asp Thr Ala Tyr

65                  70                  75                  80

Ser Gln Lys Phe Lys Gly Lys Ala Thr Leu Thr Ala Asp Lys Ser Ser
                85                  90                  95

Ser Thr Ala Tyr Met Glu Leu Arg Ser Leu Thr Ser Glu Asp Ser Ala
            100                 105                 110

Val Tyr Tyr Cys Thr Arg Phe Tyr Ser Tyr Thr Tyr Trp Gly Gln Gly
        115                 120                 125

Thr Leu Val Thr Val Ser Gly Gly Gly Gly Ser Gly Gly Gly Gly Ser
    130                 135                 140

Gly Gly Gly Gly Ser Asp Val Val Met Thr Gln Thr Pro Leu Ser Leu
145                 150                 155                 160

Pro Val Ser Leu Gly Asp Gln Ala Ser Ile Ser Cys Arg Ser Ser Gln
                165                 170                 175

Ser Leu Val His Ser Asn Gly Asn Thr Tyr Leu His Trp Tyr Leu Gln
            180                 185                 190

Lys Pro Gly Gln Ser Pro Lys Leu Leu Ile Tyr Lys Val Ser Asn Arg
        195                 200                 205

Phe Ser Gly Val Pro Asp Arg Phe Ser Gly Ser Gly Ser Gly Thr Asp
    210                 215                 220

Phe Thr Leu Lys Ile Ser Arg Val Glu Ala Glu Asp Leu Gly Val Tyr
225                 230                 235                 240

Phe Cys Ser Gln Asn Thr His Val Pro Pro Thr Phe Gly Ser Gly Thr
                245                 250                 255

Lys Leu Glu Ile Lys Arg Ala Ala Ala Phe Val Pro Val Phe Leu Pro
            260                 265                 270

Ala Lys Pro Thr Thr Thr Pro Ala Pro Arg Pro Pro Thr Pro Ala Pro
        275                 280                 285

Thr Ile Ala Ser Gln Pro Leu Ser Leu Arg Pro Glu Ala Cys Arg Pro
    290                 295                 300

Ala Ala Gly Gly Ala Val His Thr Arg Gly Leu Asp Phe Ala Cys Asp
305                 310                 315                 320

Ile Tyr Ile Trp Ala Pro Leu Ala Gly Thr Cys Gly Val Leu Leu Leu
                325                 330                 335

Ser Leu Val Ile Thr Leu Tyr Cys Asn His Arg Asn Arg Ser Lys Arg
            340                 345                 350

Ser Arg Leu Leu His Ser Asp Tyr Met Asn Met Thr Pro Arg Arg Pro
        355                 360                 365

Gly Pro Thr Arg Lys His Tyr Gln Pro Tyr Ala Pro Pro Arg Asp Phe
    370                 375                 380

Ala Ala Tyr Arg Ser Arg Phe Ser Val Val Lys Arg Gly Arg Lys Lys
385                 390                 395                 400

Leu Leu Tyr Ile Phe Lys Gln Pro Phe Met Arg Pro Val Gln Thr Thr
                405                 410                 415

Gln Glu Glu Asp Gly Cys Ser Cys Arg Phe Pro Glu Glu Glu Glu Gly
            420                 425                 430

Gly Cys Glu Leu Arg Val Lys Phe Ser Arg Ser Ala Asp Ala Pro Ala
        435                 440                 445

Tyr Gln Gln Gly Gln Asn Gln Leu Tyr Asn Glu Leu Asn Leu Gly Arg
    450                 455                 460

Arg Glu Glu Tyr Asp Val Leu Asp Lys Arg Arg Gly Arg Asp Pro Glu
465                 470                 475                 480

Met Gly Gly Lys Pro Arg Arg Lys Asn Pro Gln Glu Gly Leu Tyr Asn
                485                 490                 495

Glu Leu Gln Lys Asp Lys Met Ala Glu Ala Tyr Ser Glu Ile Gly Met
            500                 505                 510

Lys Gly Glu Arg Arg Arg Gly Lys Gly His Asp Gly Leu Tyr Gln Gly
        515                 520                 525

Leu Ser Thr Ala Thr Lys Asp Thr Tyr Asp Ala Leu His Met Gln Ala
    530                 535                 540

Leu Pro Pro Arg Glu Phe Gly Ser Gly Val Lys Gln Thr Leu Asn Phe
545                 550                 555                 560

Asp Leu Leu Lys Leu Ala Gly Asp Val Glu Ser Asn Pro Gly Pro Cys
                565                 570                 575

Met Phe His Val Ser Phe Arg Tyr Ile Phe Gly Leu Pro Pro Leu Ile
        580                 585                 590

Leu Val Leu Leu Pro Val Ala Ser Ser Asp Cys Asp Ile Glu Gly Lys
    595                 600                 605

Asp Gly Lys Gln Tyr Glu Ser Val Leu Met Val Ser Ile Asp Gln Leu
    610                 615                 620

Leu Asp Ser Met Lys Glu Ile Gly Ser Asn Cys Leu Asn Asn Glu Phe
625                 630                 635                 640

Asn Phe Phe Lys Arg His Ile Cys Asp Ala Asn Lys Glu Gly Met Phe
                645                 650                 655

Leu Phe Arg Ala Ala Arg Lys Leu Arg Gln Phe Leu Lys Met Asn Ser
            660                 665                 670

Thr Gly Asp Phe Asp Leu His Leu Leu Lys Val Ser Glu Gly Thr Thr
        675                 680                 685

Ile Leu Leu Asn Cys Thr Gly Gln Val Lys Gly Arg Lys Pro Ala Ala
    690                 695                 700

Leu Gly Glu Ala Gln Pro Thr Lys Ser Leu Glu Glu Asn Lys Ser Leu
705                 710                 715                 720

Lys Glu Gln Lys Lys Leu Asn Asp Leu Cys Phe Leu Lys Arg Leu Leu
                725                 730                 735

Gln Glu Ile Lys Thr Cys Trp Asn Lys Ile Leu Met Gly Thr Lys Glu
            740                 745                 750

His Gly Ser Gly Val Lys Gln Thr Leu Asn Phe Asp Leu Leu Lys Leu
        755                 760                 765

Ala Gly Asp Val Glu Ser Asn Pro Gly Pro Met Ala Leu Leu Leu Ala
    770                 775                 780

Leu Ser Leu Leu Val Leu Trp Thr Ser Pro Ala Pro Thr Leu Ser Gly
785                 790                 795                 800

Thr Asn Asp Ala Glu Asp Cys Cys Leu Ser Val Thr Gln Lys Pro Ile
                805                 810                 815

Pro Gly Tyr Ile Val Arg Asn Phe His Tyr Leu Leu Ile Lys Asp Gly
            820                 825                 830

Cys Arg Val Pro Ala Val Val Phe Thr Thr Leu Arg Gly Arg Gln Leu
        835                 840                 845

Cys Ala Pro Pro Asp Gln Pro Trp Val Glu Arg Ile Ile Gln Arg Leu
    850                 855                 860

Gln Arg Thr Ser Ala Lys Met Lys Arg Arg Ser Ser Gly Ser Gly Val
865                 870                 875                 880

Lys Gln Thr Leu Asn Phe Asp Leu Leu Lys Leu Ala Gly Asp Val Glu
                885                 890                 895

Ser Asn Pro Gly Pro Met Ala Ser Tyr Pro Gly His Gln His Ala Ser
            900                 905                 910

Ala Phe Asp Gln Ala Ala Arg Ser Arg Gly His Ser Asn Arg Arg Thr
        915                 920                 925

Ala Leu Arg Pro Arg Arg Gln Gln Glu Ala Thr Glu Val Arg Pro Glu
    930                 935                 940

Gln Lys Met Pro Thr Leu Leu Arg Val Tyr Ile Asp Gly Pro His Gly
945                 950                 955                 960

Met Gly Lys Thr Thr Thr Thr Gln Leu Leu Val Ala Leu Gly Ser Arg
                965                 970                 975

Asp Asp Ile Val Tyr Val Pro Glu Pro Met Thr Tyr Trp Arg Val Leu
            980                 985                 990

Gly Ala Ser Glu Thr Ile Ala Asn Ile Tyr Thr Thr Gln His Arg Leu
        995                 1000                1005

Asp Gln Gly Glu Ile Ser Ala Gly Asp Ala Ala Val Val Met Thr
    1010                1015                1020

Ser Ala Gln Ile Thr Met Gly Met Pro Tyr Ala Val Thr Asp Ala
    1025                1030                1035

Val Leu Ala Pro His Ile Gly Gly Glu Ala Gly Ser Ser His Ala
    1040                1045                1050

Pro Pro Pro Ala Leu Thr Leu Ile Phe Asp Arg His Pro Ile Ala
    1055                1060                1065

Ala Leu Leu Cys Tyr Pro Ala Ala Arg Tyr Leu Met Gly Ser Met

1070                1075                1080

Thr Pro Gln Ala Val Leu Ala Phe Val Ala Leu Ile Pro Pro Thr
    1085                1090                1095

Leu Pro Gly Thr Asn Ile Val Leu Gly Ala Leu Pro Glu Asp Arg
    1100                1105                1110

His Ile Asp Arg Leu Ala Lys Arg Gln Arg Pro Gly Glu Arg Leu
    1115                1120                1125

Asp Leu Ala Met Leu Ala Ala Ile Arg Arg Val Tyr Gly Leu Leu
    1130                1135                1140

Ala Asn Thr Val Arg Tyr Leu Gln Cys Gly Gly Ser Trp Arg Glu
    1145                1150                1155

Asp Trp Gly Gln Leu Ser Gly Thr Ala Val Pro Pro Gln Gly Ala
    1160                1165                1170

Glu Pro Gln Ser Asn Ala Gly Pro Arg Pro His Ile Gly Asp Thr
    1175                1180                1185

Leu Phe Thr Leu Phe Arg Ala Pro Glu Leu Leu Ala Pro Asn Gly
    1190                1195                1200

Asp Leu Tyr Asn Val Phe Ala Trp Ala Leu Asp Val Leu Ala Lys
    1205                1210                1215

Arg Leu Arg Ser Met His Val Phe Ile Leu Asp Tyr Asp Gln Ser
    1220                1225                1230

Pro Ala Gly Cys Arg Asp Ala Leu Leu Gln Leu Thr Ser Gly Met
    1235                1240                1245

Val Gln Thr His Val Thr Thr Pro Gly Ser Ile Pro Thr Ile Cys
    1250                1255                1260

Asp Leu Ala Arg Thr Phe Ala Arg Glu Met Gly Glu Ala Asn
    1265                1270                1275

<210>  19
<211>  3834
<212>  DNA
<213>  Artificial

<220>
<223>  (anti GPC3 scFv CAR+hIL-7+hCCL19+HSV-TK) gene

<400> 19
   atggactgga cctggaggat cctgttcctg gtggctgctg ccaccggagc tcacagcagc 60
   caggtgcagc tgcagcagag cggagctgag ctggtgagac ctggcgccag cgtgaaactg 120
   agctgcaagg ccagcggcta caccttcacc gactacgaga tgcactgggt gaagcagaca 180
   cccgtgcacg gcctgaagtg gatcggagcc ctggacccca agaccggaga caccgcctac 240
   agccagaagt ttaagggcaa ggccaccctc accgccgata agagctccag caccgcctac 300
   atggagctga ggagcctgac ctccgaggac agcgccgtgt actattgcac caggttctac 360
   agctacacct actggggcca gggaacactg gtgaccgtct ccggcggcgg cggcagcggc 420
   ggcggaggca gcggcggcgg cggcagcgat gtggtgatga cacagacccc tctgagcctg 480
   cctgtgagcc tgggagacca ggccagcatc agctgcagga gcagccagag cctggtgcac 540
   agcaacggca acacatacct ccactggtac ctgcagaagc ctggccagag ccccaagctg 600
   ctgatctaca aggtgagcaa caggttctcc ggcgtgcccg atagattcag cggctccggc 660
   agcggcaccg actttaccct gaagatctcc agagtggagg ccgaggacct gggcgtgtac 720
   ttttgcagcc agaacaccca cgtgcctcct accttcggct ccggcaccaa gctcgaaatc 780
   aagagggcgg ccgcattcgt gccggtcttc ctgccagcga agcccaccac gacgccagcg 840
   ccgcgaccac caacaccggc gcccaccatc gcgtcgcagc ccctgtccct gcgcccagag 900
   gcgtgccggc cagcggcggg gggcgcagtg cacacgaggg ggctggactt cgcctgtgat 960
   atctacatct gggcgccctt ggccgggact tgtggggtcc ttctcctgtc actggttatc 1020
   accctttact gcaaccacag gaacaggagt aagaggagca ggctcctgca cagtgactac 1080
   atgaacatga ctccccgccg ccccgggccc acccgcaagc attaccagcc ctatgcccca 1140
   ccacgcgact tcgcagccta tcgctcccgt ttctctgttg ttaaacgggg cagaaagaag 1200
   ctcctgtata tattcaaaca accatttatg agaccagtac aaactactca agaggaagat 1260
   ggctgtagct gccgatttcc agaagaagaa gaaggaggat gtgaactgag agtgaagttc 1320
   agcaggagcg cagacgcccc cgcgtaccag cagggccaga accagctcta taacgagctc 1380
   aatctaggac gaagagagga gtacgatgtt ttggacaaga gacgtggccg ggaccctgag 1440
   atggggggaa agccgagaag gaagaaccct caggaaggcc tgtacaatga actgcagaaa 1500
   gataagatgg cggaggccta cagtgagatt gggatgaaag gcgagcgccg gaggggcaag 1560
   gggcacgatg gcctttacca gggtctcagt acagccacca aggacaccta cgacgccctt 1620
   cacatgcagg ccctgccccc tcgcgaattc ggaagcggag tgaaacagac tttgaatttt 1680
   gaccttctca agttggcggg agacgtggag tccaaccctg gaccatgcat gttccatgtg 1740
   agcttcaggt acatcttcgg actgcctcct ctcatcctgg tcctcctccc cgtggccagc 1800
   tccgactgtg acatcgaagg aaaggatggc aagcagtacg aaagcgtgct gatggtgagc 1860
   atcgatcagc tcctggattc catgaaggaa atcggctcca actgcctcaa caatgagttc 1920
   aactttttta agaggcatat ctgcgacgcc aacaaggagg gcatgtttct gttcagggcc 1980
   gccaggaagc tgagacagtt cctcaagatg aatagcaccg gcgacttcga cctccatctg 2040
   ctgaaggtgt ccgagggaac caccatcctg ctgaactgca ccggccaagt gaagggaaga 2100
   aaacctgctg ccctgggcga ggctcagcct accaagagcc tcgaggagaa caaaagcctg 2160
   aaggagcaga agaagctgaa cgacctgtgc ttcctcaaga ggctcctgca ggagattaag 2220
   acctgttgga acaagatcct gatgggcaca aaggagcacg gatccggcgt gaagcagacc 2280
   ctgaactttg acctgctcaa actggccggc gacgtcgagt ccaatcctgg acctatggct 2340
   ctgctgctcg ccctgagcct gctcgtcctc tggacctccc ctgctcctac cctgagcggc 2400
   accaatgacg ctgaagactg ctgcctgtcc gtgacccaga agcctatccc cggatatatc 2460
   gtgaggaatt ttcattacct cctgatcaag gacggctgta gagtgcccgc cgtcgtgttc 2520
   acaacactca gaggcaggca gctgtgtgct ccccccgacc agccttgggt ggagagaatc 2580
   attcagagac tgcaaaggac ctccgctaag atgaagagga ggtccagcgg cagcggagtg 2640
   aagcagacac tgaatttcga cctgctcaag ctggccggcg atgtggagag caaccctgga 2700
   cctatggctt cctaccccgg acatcagcac gcttccgcct tcgaccaggc cgctagaagc 2760
   agaggacact ccaatagaag gacagccctg aggcctagga gacagcagga ggccaccgag 2820
   gtgaggcccg agcagaaaat gcccaccctg ctgagagtgt atattgatgg accccacggc 2880
   atgggaaaaa ccaccacaac ccagctgctg gtggctctgg gaagcaggga tgatattgtg 2940
   tacgtccccg aacctatgac atattggagg gtcctcggcg cctccgagac catcgccaac 3000
   atttacacca cccagcacag gctggatcag ggagagatct ccgccggcga tgctgccgtg 3060
   gtgatgacca gcgcccagat cactatgggt atgccttatg ccgtgaccga cgctgtgctg 3120
   gctcctcaca ttggcggcga agccggatcc tcccatgctc cccctcctgc cctcacactg 3180
   atctttgaca gacatcctat cgccgctctg ctgtgctacc ccgccgctag gtacctgatg 3240
   ggcagcatga cccctcaggc cgtgctggct tttgtggccc tcattccccc cacactgcct 3300
   ggcacaaata tcgtgctcgg cgccctgcct gaggacaggc acatcgatag gctggctaag 3360
   agacagagac ccggagagag gctggatctc gctatgctgg ccgccatcag gagggtgtac 3420
   ggcctgctgg ccaacaccgt gagatatctc cagtgtggcg gatcctggag ggaagactgg 3480
   ggccaactga gcggcacagc tgtgcctcct caaggcgctg agccccagag caacgctgga 3540
   cccagacctc acatcggcga taccctgttc accctgttta gagcccctga gctcctggcc 3600
   cctaacggcg acctgtacaa tgtgttcgct tgggccctgg atgtgctcgc caagagactc 3660
   aggagcatgc acgtcttcat tctggactac gaccagtccc ccgctggctg cagagatgcc 3720
   ctgctccagc tgacctccgg catggtgcag acccacgtga ccacccctgg aagcatcccc 3780
   acaatctgcg acctggccag gacctttgcc agagaaatgg gagaagccaa ctga 3834

## Description

### Technical Field

The present invention relates to a chimeric antigen receptor (which will be hereinafter referred to also as "CAR") containing a single-chain antibody that specifically binds to a human GPC3 (Glypican-3)-derived polypeptide consisting of the amino acid sequence shown by SEQ ID No: 11, a transmembrane region fused to the carboxyl (C) terminus of the single-chain antibody, and an immunocompetent cell activation signaling transduction region fused to the C-terminus of the transmembrane region; an immunocompetent cell expressing the CAR; an anticancer agent containing the immunocompetent cell; a CAR gene encoding the CAR; and a vector containing the CAR gene.

### Background Art

A CAR is an artificial chimeric protein obtained by fusing a single-chain antibody that recognizes a cell surface antigen of a cancer cell to a signaling transduction region that induces activation of T cells. A large amount of CAR-expressing T cells (which will be hereinafter referred to also as "CAR-T cells") that exhibit tumor reactivity can be produced by introducing a gene encoding the CAR into peripheral blood T cells (peripheral blood T lymphocytes) that do not exhibit tumor reactivity. The CAR-T cells that exhibit tumor reactivity have cytotoxic activity on cancer cells, independently of their interaction with major histocompatibility complex (MHC).

Clinical studies on cancer immunotherapy by administration of CAR-T cells, more specifically, therapy (Non Patent Document 1) in which a T cell is collected from a patient, a gene encoding a CAR is introduced into the T cell, and the cell is amplified and transferred into the patient again have been currently in progress all over the world. The results showing efficacy in hematopoietic malignancies such as leukemia and lymphoma have been obtained.

In recent years, various CAR-T cells have been studied. For example, there have been proposed: a pharmaceutical composition that contains a modified autologous human T cell containing a nucleic acid encoding a CAR consisting of a CD19 antigen-binding region, a transmembrane region, a 4-1BB co-stimulation signal region, and a CD3ζ signal region (Patent Document 1) ; an anti-tag chimeric antigen receptor (AT-CAR)-expressing T-cell population that is administered to a subject simultaneously with or separately from a formulation containing one or a plurality of tagged proteins configured to bind to cancer cells and is effective for one or a plurality of therapies by binding to the tagged proteins to induce cancer cell death (Patent Document 2); a cell that contains a nucleic acid encoding a chimeric antigen receptor containing an antigen-binding domain of human antibody 139, an extracellular hinge domain, a transmembrane domain, and an intracellular T cell-signaling domain (Patent Document 3); a cell that contains a nucleic acid sequence encoding a chimeric antigen receptor, wherein the chimeric antigen receptor contains an antigen-binding domain, a transmembrane domain, a co-stimulation signaling transduction region, and a CD3ζ signaling domain containing a specific amino acid sequence (Patent Document 4); a genetically engineered CD19-specific T cell that expresses and has a CD19-specific chimeric receptor on its cell surface membrane, wherein the chimeric receptor is composed of an intracellular signaling domain for the effector function of immune cells, at least one transmembrane domain, and at least one extracellular domain, and the extracellular domain contains the CD19-specific receptor (Patent Document 5); and a chimeric antigen receptor-expressing cell in which a nucleic acid encoding a chimeric antigen receptor containing the intracellular domain of a glucocorticoid-induced tumor necrosis factor receptor (GITR) as an intracellular domain is introduced (Patent Document 6) . Further, the inventors have recently reported that immune-inducing effects and antitumor activities superior to conventional CAR-T cells are exerted by a CAR-T cell expressing interleukin 7 (IL-7) or chemokine ligand 19 (CCL19) (Patent Document 7).

Meanwhile, GPC3 is an extracellular matrix protein expressed in fetal tissues, especially in the liver and kidneys, and involved in organ formation. Although GPC3 is not expressed in adult tissues other than the placenta, it is expressed in various cancer tissues such as hepatocellular carcinoma, melanoma, clear ovarian cell carcinoma, and squamous cell lung cancer. As described above, GPC3 is a protein expressed in fetal tissues like proteins, such as α-fetoprotein (AFP) and carcinoembryonic antigen (CEA), and is therefore categorized as a fetal cancer antigen. That is, GPC3 is not expressed in normal tissue cells, but it has a characteristic of being specifically expressed in cancer cells and is therefore useful as a target molecule for cancer treatment, a tumor marker, and a diagnostic marker.

As a representative of existing anti-GPC3 antibodies, there is a monoclonal antibody 1G12, available from BioMosaics Inc. This antibody is an antibody obtained by immunizing a Balb/c mouse with an antigen (polypeptide of 70 residues on the C-terminal side of GPC3) designed to avoid the complex structure and localization of GPC3 to produce a hybridoma, followed by screening with the antigen. Further, antibodies GC33 and GC199 developed by a domestic pharmaceutical manufacturer are also monoclonal antibodies established based on the same concept and are antibodies obtained by using the C-terminal partial fragment of GPC3 as an antigen (Patent Document 8).

Recently, the inventors have found an anti-GPC3 antibody that recognizes an epitope different from those recognized by existing antibodies (such as GC33 and GC199) and can specifically bind to GPC3 localized in the cell membrane even in the state of a single-chain antibody (PCT/JP2018/257).

### Prior Art Documents

### Patent Documents

Patent Document 1: U.S. Patent Application Publication No. 2014/0106449
Patent Document 2: Japanese unexamined Patent Application Publication (Translation of PCT Application) No. 2014-504294
Patent Document 3: Japanese unexamined Patent Application Publication (Translation of PCT Application) No. 2014-516510
Patent Document 4: Japanese unexamined Patent Application Publication (Translation of PCT Application) No. 2014-507118
Patent Document 5: Japanese unexamined Patent Application Publication No. 2011-004749
Patent Document 6: International Publication No. 2013/051718 Pamphlet
Patent Document 7: International Publication No. 2016/056228 Pamphlet
Patent Document 8: Japanese Patent No. 4011100

### Non-patent Documents

[Non-patent Document 1] Yozo Nakazawa, The Shinshu Medical Journal 61 (4): 197 to 203 (2013)

### Summary of the Invention

### Object to be Solved by the Invention

It is an object of the present invention to provide: a CAR that contains a single-chain antibody capable of specifically binding to GPC3 localized in the cell membrane and is useful for cancer immunotherapy using CAR-T cells; and an immunocompetent cell expressing such a CAR.

### Means to Solve the Object

The inventors are continuing diligent research to achieve the above object. In the process, they have found that a CAR-T cell with excellent cytotoxic activity on cancer cells and interferon gamma (IFN-γ) producibility can be produced by producing an anti-GPC3 single-chain antibody based on an anti-GPC3 antibody (GC33 antibody) developed by a domestic pharmaceutical manufacturer and introducing a vector that expresses a CAR to which the anti-GPC3 single-chain antibody is fused into a T cell, thereby accomplishing the present invention.

That is, the present invention is as follows.
[1] A chimeric antigen receptor (CAR) (which may be hereinafter referred to as "the CAR of the present invention") comprising: a single-chain antibody that specifically binds to a human GPC3 (Glypican-3)-derived polypeptide consisting of the amino acid sequence shown by SEQ ID No: 11; a transmembrane region fused to a carboxyl terminus of the single-chain antibody; and an immunocompetent cell activation signaling transduction region fused to the carboxyl terminus of the transmembrane region, wherein the single-chain antibody comprises: a heavy chain complementarity determining region (CDR) 1 consisting of the amino acid sequence shown by SEQ ID No: 1, a heavy-chain CDR2 consisting of the amino acid sequence shown by SEQ ID No: 2, and a heavy-chain CDR3 consisting of the amino acid sequence shown by SEQ ID No: 3; and a light-chain CDR1 consisting of the amino acid sequence shown by SEQ ID No: 4, a light-chain CDR2 consisting of the amino acid sequence shown by SEQ ID No: 5, and a light-chain CDR3 consisting of the amino acid sequence shown by SEQ ID No: 6.
[2] The CAR according to [1] above, wherein the single-chain antibody comprises: a heavy-chain variable region consisting of an amino acid sequence having at least 80% or more sequence identity with the amino acid sequence shown by SEQ ID No: 7; and a light-chain variable region consisting of an amino acid sequence having at least 80% or more sequence identity with the amino acid sequence shown by SEQ ID No: 8.
[3] The CAR according to [1] or [2] above, wherein the single-chain antibody comprises: an amino acid sequence having at least 80% or more sequence identity with the amino acid sequence shown by SEQ ID No: 12; or an amino acid sequence having at least 80% or more sequence identity with the amino acid sequence shown by SEQ ID No: 13.
[4] The CAR according to any one of [1] to [3] above, wherein the transmembrane region and the immunocompetent cell activation signaling transduction region fused to the carboxyl terminus of the transmembrane region comprise an amino acid sequence having at least 80% or more sequence identity with an amino acid sequence shown by any one of SEQ ID Nos: 14 to 16.
[5] An immunocompetent cell (which may be hereinafter referred to as "the immunocompetent cell of the present invention") expressing the CAR according to any one of [1] to [4] above.
[6] The immunocompetent cell according to [5] above, further expressing interleukin 7 (IL-7) and chemokine ligand 19 (CCL19).
[7] An anticancer agent (which may be hereinafter referred to as "the anticancer agent of the present invention") comprising: the immunocompetent cell according to [5] or [6] above; and a pharmaceutically acceptable additive.
[8] A CAR gene (which may be hereinafter referred to as "the CAR gene of the present invention") encoding the CAR according to any one of [1] to [4] above.
[9] A vector (which may be hereinafter referred to as "the CAR-expressing vector of the present invention") comprising: a promoter; and the CAR gene encoding the CAR according to [8] above operably linked downstream of the promoter.

Further, examples of other embodiments of the present invention can include a method for producing an immunocompetent cell expressing the CAR of the present invention, the method including a step of introducing the CAR-expressing vector of the present invention into an immunocompetent cell; an immunocompetent cell expressing the CAR of the present invention produced by introducing the CAR-expressing vector of the present invention into an immunocompetent cell; and an immunocompetent cell containing the CAR gene of the present invention and expressing the CAR of the present invention (preferably, an immunocompetent cell further containing IL-7 gene and CCL19 gene and expressing the CAR of the present invention, IL-7, and CCL19).

### Effect of the Invention

According to the present invention, when the CAR of the present invention is expressed on the cell membrane of an immunocompetent cell such as a T cell, the immunocompetent cell exhibits excellent cytotoxic activity on cancer cells and IFN-γ producibility. Therefore, the CAR of the present invention is useful for cancer immunotherapy using an immunocompetent cell.

### Brief Description of Drawings

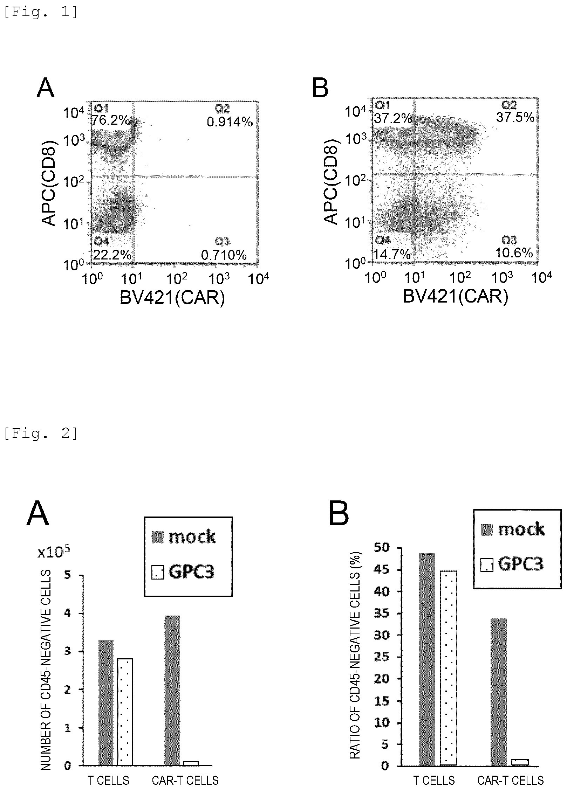

[Figure 1] Figure 1 includes graphs showing the analysis results for expression of CAR (horizontal axis) and CD8 (vertical axis) in T cells (Figure 1A) and CAR-T cells (Figure 1B) with a flow cytometer. The regions "Q1", "Q2", "Q3", and "Q4" surrounded by rectangles in the figures respectively represent a "CAR-negative CD8-positive T-cell group", a "CAR-positive CD8-positive T-cell group", a "CAR-positive CD8-negative T-cell group", and a "CAR-negative CD8-negative T-cell group", and the ratio (%) of the number of cells in each of the four cell groups with respect to the total number of cells is shown in the each region.
[Figure 2] Figure 2 shows the analysis results of the number of CD45-negative cells (corresponding to the remaining Sk-HEP-1 GPC3 cells)
(Figure 2A), and the ratio of the CD45-negative cells with respect to the total cells (Figure 2B), when Sk-HEP-1 cells ("mock" in the figures) and Sk-HEP-1 GPC3 cells ("GPC3" in the figures) are cultured in the presence of a T-cell group ("T cells" in the figures) or a T-cell group containing anti-GPC3 scFv CAR-T cells ("CAR-T cells" in the figures).

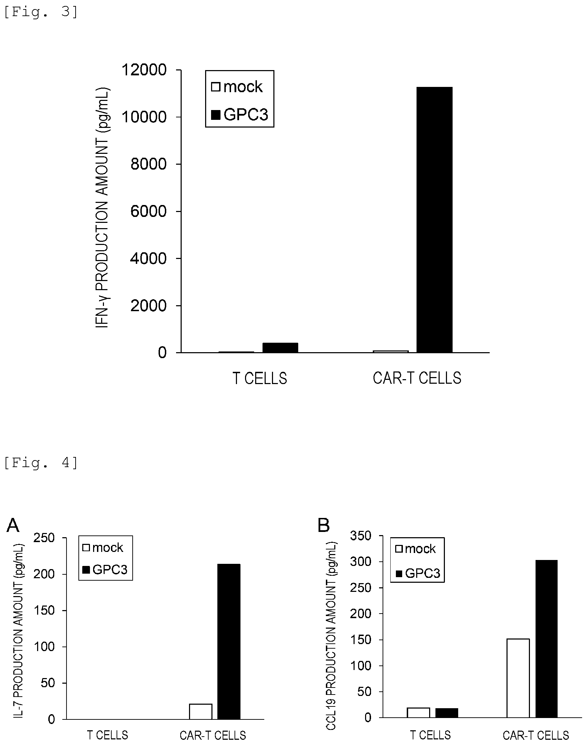

[Figure 3] Figure 3 is a graph showing the analysis results for the concentration of IFN-γ produced in a culture medium when Sk-HEP-1 cells ("mock" in the figures) and Sk-HEP-1 GPC3 cells ("GPC3" in the figures) are cultured in the presence of a T-cell group ("T cells" in the figures) or a T-cell group containing anti-GPC3 scFv CAR-T cells ("CAR-T cells" in the figures).
[Figure 4] Figure 4 includes graphs showing the analysis results for the concentration of IL-7 (Figure 4A) and CCL19 (Figure 4B) produced in a culture medium, when Sk-HEP-1 cells ("mock" in the figures) and Sk-HEP-1 GPC3 cells ("GPC3" in the figures) are cultured in the presence of a T-cell group ("T cells" in the figures) or a T-cell group containing anti-GPC3 scFv CAR-T cells ("CAR-T cells" in the figures).

### Mode of Carrying Out the Invention

The CAR of the present invention is a polypeptide containing at least i)-iii) below: i) a single-chain antibody (single-chain Fv; scFv) (which may be hereinafter referred to as "the single-chain antibody of the present invention") that contains heavy (H)-chain complementarity determining region (CDR) 1 consisting of the amino acid sequence shown by SEQ ID No: 1, H-chain CDR2 consisting of the amino acid sequence shown by SEQ ID No: 2, and H-chain CDR3 consisting of the amino acid sequence shown by SEQ ID No: 3, and light (L)-chain CDR1 consisting of the amino acid sequence shown by SEQ ID No: 4, L-chain CDR2 consisting of the amino acid sequence shown by SEQ ID No: 5, and L-chain CDR3 consisting of the amino acid sequence shown by SEQ ID No: 6, and specifically binds to a human GPC3-derived polypeptide consisting of the amino acid sequence shown by SEQ ID No: 11 (corresponding to the polypeptide consisting of glycine at position 537 to lysine at position 563 of human GPC3); ii) a transmembrane region fused to the C-terminus of the single-chain antibody of the present invention; and iii) an immunocompetent cell activation signaling transduction region fused to the C-terminus of the transmembrane region, wherein the single-chain antibody of the present invention and the transmembrane region, or the transmembrane region and the immunocompetent cell activation signaling transduction region may be fused to each other via a peptide linker or IgG4 hinge region. Further, the single-chain antibody of the present invention generally contains H-chain variable (V) regions containing the H-chain CDR1 to CDR3, L-chain V-regions containing the L-chain CDR1 to CDR3, and the H-chain V-regions and the L-chain V-regions are generally bound to each other via a peptide linker.

Examples of the length of the peptide linker can include 1 to 100 amino acid residues, preferably 10 to 50 amino acid residues. Further, specific examples of the peptide linker in the antibody of the present invention can include three continuous amino acid sequences each consisting of one to four glycines (G) and one serine (S).

In the V-region of the single-chain antibody of the present invention, framework regions (FR) are included as regions other than CDR1 to CDR3. Among such FRs, examples of H-chain FRs can include H-chain FR1 linked to the N-terminus of the H-chain CDR1, H-chain FR2 linked to the C-terminus of the H-chain CDR1 (N-terminus of the H-chain CDR2), H-chain FR3 linked to the C-terminus of the H-chain CDR2 (N-terminus of the H-chain CDR3), and H-chain FR4 linked to the C-terminus of the H-chain CDR3. Among the FRs, examples of L-chain FRs can include L-chain FR1 linked to the N-terminus of the L-chain CDR1, L-chain FR2 linked to the C-terminus of the L-chain CDR1 (N-terminus of the L-chain CDR2), L-chain FR3 linked to the C-terminus of the L-chain CDR2 (N-terminus of the L-chain CDR3), and L-chain FR4 linked to the C-terminus of the L-chain CDR3.

Examples of the H-chain FR1 can specifically include a polypeptide consisting of the amino acid residues at positions 1 to 30 in the amino acid sequence shown by SEQ ID No: 7, or a polypeptide consisting of an amino acid sequence having at least 80% or more sequence identity with the amino acid sequence of the polypeptide.

Examples of the H-chain FR2 can specifically include a polypeptide consisting of the amino acid residues at positions 36 to 49 in the amino acid sequence shown by SEQ ID No: 7, or a polypeptide consisting of an amino acid sequence having at least 80% or more sequence identity with the amino acid sequence of the polypeptide.

Examples of the H-chain FR3 can specifically include a polypeptide consisting of the amino acid residues at positions 67 to 98 in the amino acid sequence shown by SEQ ID No: 7, or a polypeptide consisting of an amino acid sequence having at least 80% or more sequence identity with the amino acid sequence of the polypeptide.

Examples of the H-chain FR4 can specifically include a polypeptide consisting of the amino acid residues at positions 105 to 114 in the amino acid sequence shown by SEQ ID No: 7, or a polypeptide consisting of an amino acid sequence having at least 80% or more sequence identity with the amino acid sequence of the polypeptide.

Examples of the L-chain FR1 can specifically include a polypeptide consisting of the amino acid residues at positions 1 to 23 in the amino acid sequence shown by SEQ ID No: 8, or a polypeptide consisting of an amino acid sequence having at least 80% or more sequence identity with the amino acid sequence of the polypeptide.

Examples of the L-chain FR2 can specifically include a polypeptide consisting of the amino acid residues at positions 40 to 54 in the amino acid sequence shown by SEQ ID No: 8, or a polypeptide consisting of an amino acid sequence having at least 80% or more sequence identity with the amino acid sequence of the polypeptide.

Examples of the L-chain FR3 can specifically include a polypeptide consisting of the amino acid residues at positions 62 to 93 in the amino acid sequence shown by SEQ ID No: 8, or a polypeptide consisting of an amino acid sequence having at least 80% or more sequence identity with the amino acid sequence of the polypeptide.

Examples of the L-chain FR4 can specifically include a polypeptide consisting of the amino acid residues at positions 103 to 113 in the amino acid sequence shown by SEQ ID No: 8, or a polypeptide consisting of an amino acid sequence having at least 80% or more sequence identity with the amino acid sequence of the polypeptide.

As the FRs of the single-chain antibody of the present invention, FRs of known human antibodies are preferable. Examples of the "FRs of known human antibodies" can include FRs of human antibodies registered in known sequence databases such as GenBank, and FRs selected from consensus sequences derived from each subgroup of human antibodies (Human Most Homologous Consensus Sequence; Kabat, E. A. et al., Sequences of Proteins of Immunological Interest, US Dept. Health and Human Services, 1991).

The H-chain CDR1 in the single-chain antibody of the present invention is generally located at positions H31 to 35 according to the numbering by Kabat (see Literature "Kabat, E.A. et al. , (1991) NIH Publication No. 91-3242, sequences of proteins of immunological interest"). Further, the H-chain CDR2 in the single-chain antibody of the present invention is generally located at positions H50 to 52, H52A, and H53 to 65 according to the numbering by Kabat. Further, the H-chain CDR3 in the single-chain antibody of the present invention is generally located at positions H95 to 100, H100A, H100B, H101, and H102 according to the numbering by Kabat. Further, the L-chain CDR1 in the single-chain antibody of the present invention is generally located at positions L24 to 34 according to the numbering by Kabat. Further, the L-chain CDR2 in the single-chain antibody of the present invention is generally located at positions L50 to 56 according to the numbering by Kabat. Further, the L-chain CDR3 in the single-chain antibody of the present invention is generally located at positions L89 to 97 according to the numbering by Kabat.

Examples of the H-chain V-region in the single-chain antibody of the present invention can specifically include a H-chain V-region consisting of an amino acid sequence having at least 80% or more sequence identity with the amino acid sequence shown by SEQ ID No: 7. Examples of the L-chain V-region in the single-chain antibody of the present invention can specifically include a L-chain V-region consisting of an amino acid sequence having at least 80% or more sequence identity with the amino acid sequence shown by SEQ ID No: 8.

Examples of the single-chain antibody of the present invention can specifically include those having the effects demonstrated in Examples described below, that is, a single-chain antibody containing an amino acid sequence having at least 80% or more sequence identity with the amino acid sequence shown by SEQ ID No: 12, and a single-chain antibody containing an amino acid sequence having at least 80% or more sequence identity with the amino acid sequence shown by SEQ ID No: 13.

The transmembrane region may be any peptide capable of penetrating through cell membranes, and examples thereof can include all or a part of a transmembrane region derived from CD8, α or β chain of a T-cell receptor, CD3ζ, CD28, CD3ε, CD45, CD4, CD5, CD8, CD9, CD16, CD22, CD33, CD37, CD64, CD80, CD86, CD134, CD137, ICOS, CD154, EGFR (Epidermal Growth Factor Receptor), or GITR. Examples thereof can specifically include a human CD8 transmembrane region consisting of the amino acid residues at positions 1 to 83 in the amino acid sequence shown by SEQ ID No: 14. Further, the transmembrane region may be those in which 1 to 10 amino acid residues, preferably 6 to 7 amino acid residues, on the C-terminal side of the peptide capable of penetrating through cell membranes are deleted. Examples thereof can include variant 1 of the human CD8 transmembrane region consisting of the amino acid residues at positions 1 to 77 in the amino acid sequence shown by SEQ ID No: 14, and variant 2 of the human CD8 transmembrane region consisting of the amino acid residues at positions 1 to 76 in the amino acid sequence shown by SEQ ID No: 14.

The immunocompetent cell activation signaling transduction region may be any region capable of transmitting signals into an immunocompetent cell when the single-chain antibody of the present invention is bound to human GPC3. Those containing at least one or more selected from polypeptides in the intracellular regions of CD28, 4-1BB (CD137), GITR, CD27, OX40, HVEM, CD3ζ, and Fc Receptor-associated γ-chain are preferable, and those containing three polypeptides in the intracellular regions of CD28, 4-1BB, and CD3ζ are more preferable. Examples of the polypeptide in the intracellular region of CD28 can specifically include a polypeptide in the intracellular region of human CD28 consisting of the amino acid residues at positions 85 to 124 in the amino acid sequence shown by SEQ ID No: 14. Examples of the "polypeptide in the intracellular region of 4-1BB" can specifically include a polypeptide in the intracellular region of human 4-1BB consisting of the amino acid residues at positions 125 to 170 in the amino acid sequence shown by SEQ ID No: 14. Examples of the polypeptide in the intracellular region of CD3ζ can specifically include a polypeptide in the intracellular region of human CD3ζ consisting of the amino acid residues at positions 172 to 283 in the amino acid sequence shown by SEQ ID No: 14.

Here, arginine (Arg) at position 84 in the amino acid sequence shown by SEQ ID No: 14, arginine at position 78 in the amino acid sequence shown by SEQ ID No: 15, and arginine at position 77 in the amino acid sequence shown by SEQ ID No: 16 are consensus sequences of the polypeptides in the transmembrane region derived from human CD8 and the polypeptides in the intracellular region of human CD28. Further, leucine (Leu) at position 171 in the amino acid sequence shown by SEQ ID No: 14, leucine at position 165 in the amino acid sequence shown by SEQ ID No: 15, and leucine at position 164 in the amino acid sequence shown by SEQ ID No: 16 are consensus sequences of the polypeptides in the intracellular region of human 4-1BB and the polypeptides in the intracellular region of human CD3ζ.

In this description, the "immunocompetent cell" means a cell responsible for the immune function in a living body (preferably, a cell separated from the living body). Examples of the immunocompetent cell can include a lymphocytic cell such as a T cell, a natural killer cell (NK cell), and a B cell; an antigen-presenting cell such as a monocyte, a macrophage, and a dendritic cell; and a granulocyte such as a neutrophil, an eosinophil, a basophil, and a mast cell. Examples thereof can specifically include a T cell derived from a mammal such as a human, a dog, a cat, a pig, and a mouse. Among them, a human-derived T cell can be mentioned as a suitable example.
Further, the T cell can be obtained, for example, by separating a cell population containing an immunocompetent cell from a body fluid such as blood and bone marrow fluid, a tissue such as spleen, thymus, and lymph nodes, or a cancer tissue such as a primary tumor, a metastatic tumor, and cancer ascites.

For increasing the proportion of the T cell contained in the cell population, the T cell can be obtained, for example, also by further isolating or purifying the cell population separated above, as required, according to a conventional method. Further, a T cell produced from an ES cell (embryonic stem cell) or an iPS cell (induced pluripotent stem cell) may be used. Examples of the T cell can include an alpha/beta T cell, a gamma/delta T cell, a CD8⁺ T cell, a CD4⁺ T cell, a tumor-infiltrating T cell, a memory T cell, a naive T cell, and an NK T cell. The origin of the immunocompetent cell and the administration target is the same or different. Further, in the case where the administration target is a human, the immunocompetent cell may be an autologous cell collected from the patient who is the administration target or an allogeneic cell collected from another person. That is, the donor and recipient may or may not match, but they preferably match.

Examples of the administration target can suitably include a mammal or a mammalian cell. Among such mammals, examples thereof can include more suitably a human, a mouse, a dog, a rat, a guinea pig, a rabbit, a sheep, a pig, a cow, a horse, a cat, a monkey, and a chimpanzee, particularly suitably a human.

The CAR of the present invention is preferably used in cancer treatment to express ex vivo on the cell surface of an immunocompetent cell collected from a cancer patient. In the case of using a T cell as the immunocompetent cell, examples of the peptide consisting of the transmembrane region in the CAR of the present invention and the immunocompetent cell activation signaling transduction region fused to the C-terminus of the transmembrane region can specifically include a peptide containing an amino acid sequence having at least 80% or more sequence identity with an amino acid sequence shown by any one of SEQ ID Nos: 14 to 16. Further, examples of the CAR of the present invention can specifically include those containing the single-chain antibody of the present invention and a peptide, linked to the C-terminal side of the single-chain antibody, containing an amino acid sequence having at least 80% or more sequence identity with the amino acid sequence of SEQ ID No: 14, those containing the single-chain antibody of the present invention and a peptide, linked to the C-terminal side of the single-chain antibody, containing an amino acid sequence having at least 80% or more sequence identity with the amino acid sequence of SEQ ID No: 15, and those containing the single-chain antibody of the present invention and a peptide, linked to the C-terminal side of the single-chain antibody, containing an amino acid sequence having at least 80% or more sequence identity with the amino acid sequence of SEQ ID No: 16.

The immunocompetent cell of the present invention may be any immunocompetent cell that expresses the CAR of the present invention, and since the CAR of the present invention usually does not exist naturally, it is an immunocompetent cell that expresses an exogenous CAR rather than an endogenous CAR. The immunocompetent cell of the present invention further preferably express IL-7 and/or CCL19. In the case where the immunocompetent cell is a cell that does not express IL-7 and/or CCL19 (such as a T cell), or the immunocompetent cell is a cell other than the T cell and exhibits a low expression level of IL-7 and/or CCL19, the immunocompetent cell of the present invention preferably expresses exogenous IL-7 and/or CCL19. Here, the immunocompetent cell expressing exogenous IL-7 and/or CCL19 can be obtained by introducing exogenous IL-7 gene and/or CCL19 gene (preferably, exogenous IL-7 gene and/or CCL19 gene operably linked downstream of the promoter) into an immunocompetent cell. The immunocompetent cell obtained by introducing the exogenous IL-7 gene and/or CCL19 gene contains the exogenous IL-7 gene and/or CCL19 gene incorporated into the genome or not incorporated into the genome (for example, in an episomal state) within the immunocompetent cell. The immunocompetent cell of the present invention also includes a cell culture that is obtained by culturing the immunocompetent cell of the present invention and expresses the CAR of the present invention.

The immunocompetent cell of the present invention can be produced by introducing the CAR-expressing vector of the present invention into the immunocompetent cell. The introduction method may be any method for introducing DNA into an immunocompetent cell. Examples thereof can include methods such as the electroporation method (Cytotechnology, 3, 133 (1990)), the calcium phosphate method (Japanese unexamined Patent Application Publication No. 2-227075), the lipofection method (Proc. Natl. Acad. Sci. U. S. A., 84, 7413 (1987)), and a virus infection method. Examples of the virus infection method can include a method in which a CAR-expressing vector (International Publication No. 2016/056228 Pamphlet) and a packaging plasmid are transfected into a packaging cell such as a GP2-293 cell (available from Takara Bio Inc.), a Plat-GP cell (available from Cosmo Bio Co., Ltd.), a PG13 cell (ATCC CRL-10686), and a PA317 cell (ATCC CRL-9078) to produce a recombinant virus, and a T cell is infected with the recombinant virus. The immunocompetent cell (the immunocompetent cell of the present invention) obtained by introducing the CAR-expressing vector of the present invention contains the CAR gene of the present invention incorporated into the genome or not incorporated into the genome within the immunocompetent cell.

Further, the immunocompetent cell of the present invention may be produced also by incorporating a nucleotide encoding the CAR of the present invention into the genome of the cell using a known gene-editing technique so as to be able to express under the control of an appropriate promoter. Examples of the known gene-editing technique can include a technique using an endonuclease such as zinc finger nuclease, TALEN (transcriptional activation-like effector nuclease), and CRISPR (Clustered Regularly Interspaced Short Palindromic Repeat)-Cas system.

The anticancer agent of the present invention may be any composition the application of which is specified "for anti-cancer (that is, for suppressing the growth of cancer)" and which contains the immunocompetent cell of the present invention and a pharmaceutically acceptable additive. Examples of the pharmaceutically acceptable additive can include normal saline, buffer normal saline, cell culture medium, dextrose, water for injection, glycerol, ethanol, a stabilizer, a solubilizer, a surfactant, a buffer, a preservative, an isotonic agent, a filler, and a lubricant. Further, the additive may be a simple substance consisting of one additive or a mixture containing a plurality (at least two) of additives.

The anticancer agent of the present invention can be administered to a test subject (cancer patient) in need of suppression of cancer growth (in other words, cancer treatment) using a method known to those skilled in the art. Examples of the administration method can include a method of intravenous, intratumor, intradermal, subcutaneous, intramuscular, intraperitoneal, intraarterial, intramedullary, intracardiac, intraarticular, intrasynovial, intracranial, intrathecal, or subarachnoid (spinal fluid) injection and an administration method to a tumor site using a catheter.

The number of immunocompetent cells of the present invention to be contained in the anticancer agent of the present invention can be appropriately selected depending on the conditions such as the type, location, and severity of cancer, and the age and body weight and condition of the subject to be treated. For example, the number of cells is 1 × 10⁴ to 1 × 10¹⁰, preferably 1 × 10⁵ to 1 × 10⁹, more preferably 5 × 10⁶ to 5 × 10⁸ per administration.

The number of times of administration of the anticancer agent of the present invention is, for example, 4 times, 3 times, twice, or once per day. Further, the administration interval of the anticancer agent of the present invention is, for example, every other day, every 3 days, every 4 days, every 5 days, every 6 days, every 7 days, every 8 days, every 9 days, every 10 days, or every other month.

Examples of the cancer for which the anticancer agent of the present invention is administered can include colorectal cancer (colon cancer or rectal cancer); gastric cancer; liver cancer; brain tumor; lung cancer (adenocarcinoma, squamous-cell carcinoma, adenosquamous carcinoma, undifferentiated cancer, large cell carcinoma, or small cell carcinoma); esophageal cancer; duodenal cancer; small intestine cancer; skin cancer; breast cancer; prostate cancer; bladder cancer; vaginal cancer; cervical cancer; endometrial cancer; renal cancer; pancreatic cancer; spleen cancer; tracheal cancer; bronchial cancer; head and neck cancer; gallbladder cancer; cholangiocarcinoma; testicular cancer; ovarian cancer; cancer in bone tissues, cartilage tissues, adipose tissues, muscle tissues, nerve tissues, vascular tissues, or hematopoietic tissues (specifically, a sarcoma such as chondrosarcoma, Ewing's sarcoma, malignant hemangioendothelioma, malignant schwannoma, osteosarcoma, or soft tissue sarcoma; a blastoma such as hepatoblastoma, medulloblastoma, nephroblastoma, neuroblastoma, pancreatoblastoma, pleuropulmonary blastoma, or retinoblastoma; embryonic cell tumor; lymphoma; or leukemia).

The anticancer agent of the present invention can be used in combination with another anticancer agent. Examples of the other anticancer agent can include an alkylating drug such as cyclophosphamide, bendamustine, iosfamide, or dacarbazine, an antimetabolite such as pentostatin, fludarabine, cladribine, methotrexate, 5-fluorouracil, 6-mercaptopurine, or enocitabin, a molecular targeted drug such as rituximab, cetuximab, or trastuzumab, a kinase inhibitor such as imatinib, gefitinib, erlotinib, afatinib, dasatinib, sunitinib, or trametinib, a proteasome inhibitor such as bortezomib, a calcineurin inhibitor such as cyclosporine or tacrolimus, an antitumor antibiotic such as idarubicin or doxorubicin mitomycin C, a plant alkaloid such as irinotecan or etoposide, a platinum preparation such as cisplatin, oxaliplatin, or carboplatin, a hormone therapeutic drug such as tamoxifen or bicalutamide, or an immunoregulatory drug such as interferon, nivolumab, or pembrolizumab.

Examples of the method of "using the anticancer agent of the present invention in combination with another anticancer agent" can include a treatment method using another anticancer agent and then the anticancer agent of the present invention, a method using the anticancer agent of the present invention and another anticancer agent at the same time, or a treatment method using the anticancer agent of the present invention and then another anticancer agent. Further, in the case of using the anticancer agent of the present invention in combination with another anticancer agent, the therapeutic effect on cancer is further improved, and each side effect can be reduced by reducing the number of doses or dose.

The CAR gene of the present invention is not specifically limited as long as it is an antibody gene (nucleotide) encoding a polypeptide containing at least i)-iii) below: i) the single-chain antibody of the present invention, ii) a transmembrane region fused to the C-terminus of the single-chain antibody of the present invention, and iii) an immunocompetent cell activation signaling transduction region fused to the C-terminus of the transmembrane region. Examples of the antibody gene encoding the single-chain antibody of the present invention can specifically include a H-chain V-region gene consisting of a nucleotide sequence having at least 80% or more sequence identity with the nucleotide sequence shown by SEQ ID No: 9 (gene encoding a H-chain V-region consisting of an amino acid sequence having at least 80% or more sequence identity with the amino acid sequence shown by SEQ ID No: 7), and a L-chain V-region gene consisting of a nucleotide sequence having at least 80% or more sequence identity with the nucleotide sequence shown by SEQ ID No: 10 (gene encoding a L-chain V-region consisting of an amino acid sequence having at least 80% or more sequence identity with the amino acid sequence shown by SEQ ID No: 8).

In this description, the term "identity" means the degree of approximation of the polypeptide or polynucleotide sequence (which is determined depending on matching between the query sequence and another sequence, preferably, of the same type (nucleic acid or protein sequence)). Examples of a preferable method of computer program for calculating and determining the "identity" can include GCG BLAST (Basic Local Alignment Search Tool) (Altschul et al., J. Mol. Biol. 1990, 215: 403-410; Altschul et al., Nucleic Acids Res. 1997, 25: 3389-3402; Devereux et al., Nucleic Acid Res. 1984, 12: 387), and BLASTN 2.0 (Gish W., http://blast.Wustl.edu, 1996-2002), and FASTA (Pearson and Lipman, Proc. Natl. Acad. Sci. USA 1988, 85: 2444-2448), and GCG GelMerge to determine and align the longest overlapping pair of contigs (Wibur and Lipman, SIAM J. Appl. Math. 1984, 44: 557-567; Needleman and Wunsch, J. Mol. Biol. 1970, 48: 443-453).

In this description, the "at least 80% or more identity" means that the identity is 80% or more, preferably 85% or more, more preferably 88% or more, further preferably 90% or more, furthermore more preferably 93% or more, particularly preferably 95% or more, still more preferably 98% or more, most preferably 100%.

In this description, the "amino acid sequence having at least 80% or more sequence identity with the amino acid sequence shown by SEQ ID No: X" is, in other words, "an amino acid sequence in which zero, one, or some amino acid residues are deleted, substituted, inserted, and/or added in the amino acid sequence shown by SEQ ID No: X" and has a function equivalent to the amino acid sequence shown by SEQ ID No: X. Here, the "amino acid sequence in which one or some amino acid residues are deleted, substituted, inserted, and/or added", for example, means an amino acid sequence in which the number of the amino acid residues deleted, substituted, inserted, and/or added is in the range of 1 to 30, preferably in the range of 1 to 20, more preferably in the range of 1 to 15, further preferably in the range of 1 to 10, further preferably in the range of 1 to 5, further preferably in the range of 1 to 3, further preferably in the range of 1 to 2. Mutation treatment of these amino acid residues can be performed by any method known to those skilled in the art, such as chemical synthesis, genetic engineering, and mutagenesis.

In this description, the "gene operably linked downstream of the promoter" means that the promoter DNA and the gene DNA are functionally linked so that the promoter can initiate gene transcription.

The promoter in the CAR-expressing vector of the present invention may be any region that initiates transcription of mRNA encoded by the CAR gene of the present invention located downstream of the promoter, and the promoter generally includes the transcription start site (TSS).

The type of the promoter or vector in the CAR-expressing vector of the present invention can be appropriately selected, corresponding to the type of the host cell (immunocompetent cell) to be introduced. Here, examples of the promoter can include cytomegalovirus (CMV) IE (immediate early) gene promoter, SV40 early promoter, retrovirus promoter, metallothionein promoter, heat shock promoter, SRα promoter, NFAT promoter, and HIF promoter. Further, examples of the vector can include a retroviral vector such as pMSGV vector (Tamada k et al., Clin Cancer Res 18: 6436-6445 (2002)) or pMSCV vector (available from Takara Bio Inc.), or those derived from such a vector.

The CAR-expressing vector of the present invention may further contain a nucleotide sequence of an enhancer region or ribosome-binding site (RBS), for further enhancing the gene expression efficiency, drug-resistant gene (such as spectinomycin-resistant gene, chloramphenicol-resistant gene, tetracycline-resistant gene, kanamycin-resistant gene, ampicillin-resistant gene, puromycin-resistant gene, hygromycin-resistant gene, blasticidin-resistant gene, and genetisin-resistant gene) corresponding to the type of the immunocompetent cell of the present invention, for screening the immunocompetent cell of the present invention. Such an enhancer region is generally arranged upstream of the promoter, and RBS is generally arranged between the promoter and the CAR gene of the present invention. In the nucleotide sequence of the CAR gene of the present invention in the CAR-expressing vector of the present invention, the codon sequence may be optimized for the expressing immunocompetent cell of the present invention. The CAR of the present invention, the CAR-expressing vector of the present invention, and the CAR gene of the present invention can be produced by known methods using the gene recombination technology.

The CAR-expressing vector of the present invention may further contain a gene other than the CAR gene of the present invention, such as IL-7 gene, CCL19 gene, or a gene encoding a polypeptide with apoptotic action (suicide gene) (preferably, IL-7 gene, CCL19 gene, or a suicide gene operably linked downstream of the promoter). Examples of the suicide gene can include a gene encoding herpes simplex virus thymidine kinase [HSV-TK] or a gene encoding inducible caspase 9. For example, in the case where cancer cells disappear in the course of cancer treatment, the CAR-expressing vector of the present invention containing a suicide gene enables the immunocompetent cells of the present invention in the body that are no longer needed to be killed by administering a drug that activates the functions of the polypeptide encoded by the suicide gene (which is ganciclovir in the case of HSV-TK or AP1903 that is chemical induction of dimerization: CID in the case of inducible caspase 9, for example) (Cooper LJ., et. al. Cytotherapy. 2006; 8 (2): 105-17., Jensen M. C. et. al. Biol Blood Marrow Transplant. 2010 Sep; 16 (9): 1245-56., Jones BS. Front Pharmacol. 2014 Nov 27; 5: 254., Minagawa K., Pharmaceuticals (Basel). 2015 May 8; 8(2): 230-49., and Bole-Richard E., Front Pharmacol. 2015 Aug 25; 6: 174) .

References such as academic literatures, patents, and patent applications cited herein are hereby incorporated by reference in their entirety to the same extent as they are specifically described in this description.

Hereinafter, the present invention will be described more specifically according to Examples, but the technical range of the present invention is not limited to these examples. In Examples below, GP2-293 packaging cell line (available from Takara Bio Inc.) was cultured in a CO₂ incubator (under the condition of 37°C) in the presence of DMEM (Dulbecco's Modified Eagle's Medium) culture medium containing 10% fetal bovine serum (FBS), penicillin G, and streptomycin sulfate. Further, the human T cells were cultured in a CO₂ incubator (under the condition of 37°C) in the presence of GT-T551 (available from Takara Bio Inc.) culture medium containing penicillin G (100 Units/mL) and others.

### Examples

### 1. Preparation of materials

### [Production of CAR, hIL-7, hCCL19, and HSV-TK-expressing retroviral vector]

Based on the amino acid sequences of a H-chain V-region (VH) and a L-chain V-region (V_{L}) of an anti-GPC3 antibody (GC33 antibody) developed by a domestic pharmaceutical manufacturer, two types of anti-GPC3 human scFvs (V_{H}-linker-V_{L} [polypeptide consisting of the amino acid sequence of SEQ ID No: 12] and V_{L}-linker-V_{H} [polypeptide consisting of the amino acid sequence of SEQ ID No: 13]) were designed (see Table 1). A linker consisting of 15 amino acid residues obtained by repeating a polypeptide "GGGGS" three times was used therefor.

**[Table 1]**

| |
|---|
| SEQ ID No: 12: Anti-GPC3 human scFvs |
| QVQLQQSGAELVRPGASVKLSCKASGYTFTDYEMHWVKQTPVHGLKWIGALDPKTGDTAYSQKFKGKA TLTADKSSSTAYMELRSLTSEDSAVYYCTRFYSYTYWGQGTLVTVS GGGGSGGGGSGGGGS DVVMTQ TPLSLPVSLGDQASISCRSSQSLVHSNGNTYLHWYLQKPGQSPKLLIYKVSNRFSGVPDRFSGSGSGT DFTLKISRVEAEDLGVYFCSQNTHVPPTFGSGTKLEIKR |
| SEQ ID No: 13: Anti-GPC3 human scFvs (Ver. 2) |
| DVVMTQTPLSLPVSLGDQASISCRSSQSLVHSNGNTYLHWYLQKPGQSPKLLIYKVSNRFSGVPDRFS GSGSGTDFTLKISRVEAEDLGVYFCSQNTHVPPTFGSGTKLEIKRGGGGSGGGGSGGGGSQVQLQQS GAELVRPGASVKLSCKASGYTFTDYEMHWVKQTPVHGLKWIGALDPKTGDTAYSQKFKGKATLTADKS SSTAYMELRSLTSEDSAVYYCTRFYSYTYWGQGTLVTVS |

In the table, the double squares each represent a linker, the single underlines each represent VH, and the double underlines each represent VL.

Of the two types of anti-GPC3 human scFvs designed, VH-linker-VL (polypeptide consisting of the amino acid sequence of SEQ ID No: 12) is selected as an example, and a fusion peptide consisting of a human CD8-derived transmembrane region and a human CD28/4-1BB/CD3zeta-derived immunocompetent cell activation signaling transduction region (the amino acid residues at positions 243 to 525 in the amino acid sequence of SEQ ID No: 17) was linked downstream thereof, to design an anti-GPC3 scFv CAR consisting of the amino acid sequence of SEQ ID No: 17 (see Table 2).

**[Table 2]**

| |
|---|
| SEQ ID No: 17: CAR of scFvs human antibody of GC33 antibody |
| QVQLQQSGAELVRPGASVKLSCKASGYTFTDYEMHWVKQTPVHGLKWIGALDPKTGDTAYSQKFKGKAT LTADKSSSTAYMELRSLTSEDSAVYYCTRFYSYTYWGQGTLVTVSGGGGSGGGGSGGGGSDVVMTQTPL SLPVSLGDQASISCRSSQSLVHSNGNTYLHWYLQKPGQSPKLLIYKVSNRFSGVPDRFSGSGSGTDFTL KISRVEAEDLGVYFCSQNTHVPPTFGSGTKLEIKRFVPVFLPAKPTTTPAPRPPTPAPTIASQPLSLRP EACRPAAGGAVHTRGLDFACDIYIWAPLAGTCGVLLLSLVITLYCNHRNRSKRSRLLHSDYMNMTPRRPG PTRKHYQPYAPPRDFAAYRSRFSVVKRGRKKLLYIFKQPFMRPVQTTQEEDGCSCRFPEEEEGGCELRVK FSRSADAPAYQQGQNQLYNELNLGRREEYDVLDKRRGRDPEMGGKPRRKNPQEGLYNELQKDKMAEAYSE IGMKGERRRGKGHDGLYQGLSTATKDTYDALHMQALPPR |

In the table, the single underline represents an anti-GPC3 human scFv, the double underline represents a fusion peptide consisting of a human CD8-derived transmembrane region and a human CD28/4-1BB/CD3zeta-derived immunocompetent cell activation signaling transduction region.

Further, a human immunoglobulin H-chain-derived signal sequence (amino acid residues at positions 1 to 19 in the amino acid sequence of SEQ ID No: 18) was linked upstream of the anti-GPC3 scFv CAR designed, and a 2A self-cleaving peptide (amino acid residues at positions 551 to 575 in the amino acid sequence of SEQ ID No: 18), human (h)IL-7 (amino acid residues at positions 577 to 753 in the amino acid sequence of SEQ ID No: 18), a 2A self-cleaving peptide (amino acid residues at positions 754 to 778 in the amino acid sequence of SEQ ID No: 18), hCCL19 (amino acid residues at positions 779 to 876 in the amino acid sequence of SEQ ID No: 18), a 2A self-cleaving peptide (amino acid residues at positions 877 to 901 in the amino acid sequence of SEQ ID No: 18), and herpes simplex virus thymidine kinase (HSV-TK) (amino acid residues at positions 902 to 1277 in the amino acid sequence of SEQ ID No: 18) were sequentially linked downstream thereof, to design a fusion polypeptide consisting of the amino acid sequence of SEQ ID No: 18 (anti-GPC3 scFv CAR+hIL-7+hCCL19+HSV-TK) (see Table 3) .

**[Table 3]**

| |
|---|
| SEQ ID No: 18: CAR+hIL-7+hCCL19+HSV-TK |
| MDWTWRILFLVAAATGAHS SQVQLQQSGAELVRPGASVKLSCKASGYTFTDYEMHWVKQTPVHGLKWIG ALDPKTGDTAYSQKFKGKATLTADKSSSTAYMELRSLTSEDSAVYYCTRFYSYTYWGQGTLVTVSGGGGS GGGGSGGGGSDVVMTQTPLSLPVSLGDQASISCRSSQSLVHSNGNTYLHWYLQKPGQSPKLLIYKVSNRF SGVPDRFSGSGSGTDFTLKISRVEAEDLGVYFCSQNTHVPPTFGSGTKLEIKRAAAFVPVFLPAKPTTTP APRPPTPAPTIASQPLSLRPEACRPAAGGAVHTRGLDFACDIYIWAPLAGTCGVLLLSLVITLYCNHRNR SKRSRLLHSDYMNMTPRRPGPTRKHYQPYAPPRDFAAYRSRFSVVKRGRKKLLYIFKQPFMRPVQTTQEE DGCSCRFPEEEEGGCELRVKFSRSADAPAYQQGQNQLYNELNLGRREEYDVLDKRRGRDPEMGGKPRRKN PQEGLYNELQKDKMAEAYSEIGMKGERRRGKGHDGLYQGLSTATKDTYDALHMQALPPREF GSGVKQTL NFDLLKLAGDVESNPGP CMFHVSFRYIFGLPPLILVLLPVASSDCDIEGKDGKQYESVLMVSIDQLLDS MKEIGSNCLNNEFNFFKRHICDANKEGMFLFRAARKLRQFLKMNSTGDFDLHLLKVSEGTTILLNCTGQV KGRKPAALGEAQPTKSLEENKSLKEQKKLNDLCFLKRLLQEIKTCWNKILMGTKEH GSGVKQTLNFDLL KLAGDVESNPGP MALLLALSLLVLWTSPAPTLSGTNDAEDCCLSVTQKPIPGYIVRNFHYLLIKDGCRV PAVVFTTLRGRQLCAPPDQPWVERIIQRLQRTSAKMKRRSS GSGVKQTLNFDLLKLAGDVESNPGP MAS YPGHQHASAFDQAARSRGHSNRRTALRPRRQQEATEVRPEQKMPTLLRVYIDGPHGMGKTTTTQLLVALG SRDDIVYVPEPMTYWRVLGASETIANIYTTQHRLDQGEISAGDAAVVMTSAQITMGMPYAVTDAVLAPHI GGEAGSSHAPPPALTLIFDRHPIAALLCYPAARYLMGSMTPQAVLAFVALIPPTLPGTNIVLGALPEDRH IDRLAKRQRPGERLDLAMLAAIRRVYGLLANTVRYLQCGGSWREDWGQLSGTAVPPQGAEPQSNAGPRPH IGDTLFTLFRAPELLAPNGDLYNVFAWALDVLAKRLRSMHVFILDYDQSPAGCRDALLQLTSGMVQTHVT TPGSIPTICDLARTFAREMGEAN |

In the table, the single square represents a human immunoglobulin H-chain-derived signal sequence, the double squares each represent a 2A self-cleaving peptide, the single underline represents a CAR, the double underline represents hIL-7, the broken underline represents hCCL19, and the wavy underline represents HSV-TK.

Then, a gene DNA consisting of a nucleotide sequence encoding the fusion polypeptide (CAR+hIL-7+hCCL19+HSV-TK) and optimized for human codon (polynucleotide consisting of the nucleotide sequence of SEQ ID No: 19, see Table 4) was incorporated into a pMSGV retroviral vector (Tamada k et al. , Clin Cancer Res 18: 6436-6445 (2002)), to produce a retroviral vector for expression of CAR, hIL-7, hCCL19, and HSV-TK (which will be hereinafter referred to as "retroviral vector for expression of anti-GPC3 scFv CAR, etc.") (see International Publication No. 2016/056228 Pamphlet).

**[Table 4]**

| |
|---|
| SEQ ID No: 19: "CAR+hIL-7+hCCL19+HSV-TK" gene |
| ATGGACTGGACCTGGAGGATCCTGTTCCTGGTGGCTGCTGCCACCGGAGCTCACAGCAGCCAGGTGCAG CTGCAGCAGAGCGGAGCTGAGCTGGTGAGACCTGGCGCCAGCGTGAAACTGAGCTGCAAGGCCAGCGGCT ACACCTTCACCGACTACGAGATGCACTGGGTGAAGCAGACACCCGTGCACGGCCTGAAGTGGATCGGAGC CCTGGACCCCAAGACCGGAGACACCGCCTACAGCCAGAAGTTTAAGGGCAAGGCCACCCTCACCGCCGAT AAGAGCTCCAGCACCGCCTACATGGAGCTGAGGAGCCTGACCTCCGAGGACAGCGCCGTGTACTATTGCA CCAGGTTCTACAGCTACACCTACTGGGGCCAGGGAACACTGGTGACCGTCTCCGGCGGCGGCGGCAGCGG CGGCGGAGGCAGCGGCGGCGGCGGCAGCGATGTGGTGATGACACAGACCCCTCTGAGCCTGCCTGTGAGC CTGGGAGACCAGGCCAGCATCAGCTGCAGGAGCAGCCAGAGCCTGGTGCACAGCAACGGCAACACATACC TCCACTGGTACCTGCAGAAGCCTGGCCAGAGCCCCAAGCTGCTGATCTACAAGGTGAGCAACAGGTTCTC CGGCGTGCCCGATAGATTCAGCGGCTCCGGCAGCGGCACCGACTTTACCCTGAAGATCTCCAGAGTGGAG GCCGAGGACCTGGGCGTGTACTTTTGCAGCCAGAACACCCACGTGCCTCCTACCTTCGGCTCCGGCACCA AGCTCGAAATCAAGAGGGCGGCCGCATTCGTGCCGGTCTTCCTGCCAGCGAAGCCCACCACGACGCCAGC GCCGCGACCACCAACACCGGCGCCCACCATCGCGTCGCAGCCCCTGTCCCTGCGCCCAGAGGCGTGCCGG CCAGCGGCGGGGGGCGCAGTGCACACGAGGGGGCTGGACTTCGCCTGTGATATCTACATCTGGGCGCCCT TGGCCGGGACTTGTGGGGTCCTTCTCCTGTCACTGGTTATCACCCTTTACTGCAACCACAGGAACAGGAG TAAGAGGAGCAGGCTCCTGCACAGTGACTACATGAACATGACTCCCCGCCGCCCCGGGCCCACCCGCAAG |
| CATTACCAGCCCTATGCCCCACCACGCGACTTCGCAGCCTATCGCTCCCGTTTCTCTGTTGTTAAACGGG GCAGAAAGAAGCTCCTGTATATATTCAAACAACCATTTATGAGACCAGTACAAACTACTCAAGAGGAAGA TGGCTGTAGCTGCCGATTTCCAGAAGAAGAAGGAGGATGTGAACTGAGAGTGAAGTTCAGCAGGAGC GCAGACGCCCCCGCGTACCAGCAGGGCCAGAACCAGCTCTATAACGAGCTCAATCTAGGACGAAGAGAGG AGTACGATGTTTTGGACAAGAGACGTGGCCGGGACCCTGAGATGGGGGGAAAGCCGAGAAGGAAGAACCC TCAGGAAGGCCTGTACAATGAACTGCAGAAAGATAAGATGGCGGAGGCCTACAGTGAGATTGGGATGAAA GGCGAGCGCCGGAGGGGCAAGGGGCACGATGGCCTTTACCAGGGTCTCAGTACAGCCACCAAGGACACCT ACGACGCCCTTCACATGCAGGCCCTGCCCCCTCGCGAATTCGGAAGCGGAGTGAAACAGACTTTGAATT TTGACCTTCTCAAGTTGGCGGGAGACGTGGAGTCCAACCCTGGACCATGCATGTTCCATGTGAGCTTCA GGTACATCTTCGGACTGCCTCCTCTCATCCTGGTCCTCCTCCCCGTGGCCAGCTCCGACTGTGACATCGA AGGAAAGGATGGCAAGCAGTACGAAAGCGTGCTGATGGTGAGCATCGATCAGCTCCTGGATTCCATGAAG GAAATCGGCTCCAACTGCCTCAACAATGAGTTCAACTTTTTTAAGAGGCATATCTGCGACGCCAACAAGG AGGGCATGTTTCTGTTCAGGGCCGCCAGGAAGCTGAGACAGTTCCTCAAGATGAATAGCACCGGCGACTT CGACCTCCATCTGCTGAAGGTGTCCGAGGGAACCACCATCCTGCTGAACTGCACCGGCCAAGTGAAGGGA AGAAAACCTGCTGCCCTGGGCGAGGCTCAGCCTACCAAGAGCCTCGAGGAGAACAAAAGCCTGAAGGAGC AGAAGAAGCTGAACGACCTGTGCTTCCTCAAGAGGCTCCTGCAGGAGATTAAGACCTGTTGGAACAAGAT CCTGATGGGCACAAAGGAGCACGGATCCGGCGTGAAGCAGACCCTGAACTTTGACCTGCTCAAACTGGC CGGCGACGTCGAGTCCAATCCTGGACCTATGGCTCTGCTGCTCGCCCTGAGCCTGCTCGTCCTCTGGAC CTCCCCTGCTCCTACCCTGAGCGGCACCAATGACGCTGAAGACTGCTGCCTGTCCGTGACCCAGAAGCCT ATCCCCGGATATATCGTGAGGAATTTTCATTACCTCCTGATCAAGGACGGCTGTAGAGTGCCCGCCGTCG TGTTCACAACACTCAGAGGCAGGCAGCTGTGTGCTCCCCCCGACCAGCCTTGGGTGGAGAGAATCATTCA GAGACTGCAAAGGACCTCCGCTAAGATGAAGAGGAGGTCCAGCGGCAGCGGAGTGAAGCAGACACTGAA TTTCGACCTGCTCAAGCTGGCCGGCGATGTGGAGAGCAACCCTGGACCTATGGCTTCCTACCCCGGACA TCAGCACGCTTCCGCCTTCGACCAGGCCGCTAGAAGCAGAGGACACTCCAATAGAAGGACAGCCCTGAGG CCTAGGAGACAGCAGGAGGCCACCGAGGTGAGGCCCGAGCAGAAAATGCCCACCCTGCTGAGAGTGTATA TTGATGGACCCCACGGCATGGGAAAAACCACCACAACCCAGCTGCTGGTGGCTCTGGGAAGCAGGGATGA TATTGTGTACGTCCCCGAACCTATGACATATTGGAGGGTCCTCGGCGCCTCCGAGACCATCGCCAACATT TACACCACCCAGCACAGGCTGGATCAGGGAGAGATCTCCGCCGGCGATGCTGCCGTGGTGATGACCAGCG CCCAGATCACTATGGGTATGCCTTATGCCGTGACCGACGCTGTGCTGGCTCCTCACATTGGCGGCGAAGC CGGATCCTCCCATGCTCCCCCTCCTGCCCTCACACTGATCTTTGACAGACATCCTATCGCCGCTCTGCTG TGCTACCCCGCCGCTAGGTACCTGATGGGCAGCATGACCCCTCAGGCCGTGCTGGCTTTTGTGGCCCTCA TTCCCCCCACACTGCCTGGCACAAATATCGTGCTCGGCGCCCTGCCTGAGGACAGGCACATCGATAGGCT GGCTAAGAGACAGAGACCCGGAGAGAGGCTGGATCTCGCTATGCTGGCCGCCATCAGGAGGGTGTACGGC CTGCTGGCCAACACCGTGAGATATCTCCAGTGTGGCGGATCCTGGAGGGAAGACTGGGGCCAACTGAGCG GCACAGCTGTGCCTCCTCAAGGCGCTGAGCCCCAGAGCAACGCTGGACCCAGACCTCACATCGGCGATAC CCTGTTCACCCTGTTTAGAGCCCCTGAGCTCCTGGCCCCTAACGGCGACCTGTACAATGTGTTCGCTTGG GCCCTGGATGTGCTCGCCAAGAGACTCAGGAGCATGCACGTCTTCATTCTGGACTACGACCAGTCCCCCG |
| CTGGCTGCAGAGATGCCCTGCTCCAGCTGACCTCCGGCATGGTGCAGACCCACGTGACCACCCCTGGAAG CATCCCCACAATCTGCGACCTGGCCAGGACCTTTGCCAGAGAAATGGGAGAAGCCAACTGA |

In the table, the single square represents a gene encoding a human immunoglobulin H-chain-derived signal sequence, the double squares each represent a gene encoding a 2A self-cleaving peptide, the single underline represents a gene encoding a CAR, the double underline represents a gene encoding hIL-7, the broken underline represents a gene encoding hCCL19, and the wavy underline represents a gene encoding HSV-TK.

### [Production of recombinant retrovirus]

The retroviral vector for expression of anti-GPC3 scFv CAR, etc. produced was transfected into a GP2-293 packaging cell line (available from Takara Bio Inc.) using Lipofectamine 3000 (available from Life Technologies Corporation). 48 hours after the transfection, a culture supernatant containing the recombinant retrovirus (retrovirus for expression of anti-GPC3 scFv CAR, etc.) produced was collected.

### [Infection of T cells with recombinant retrovirus]

In order to produce anti-GPC3 scFv CAR-expressing T cells, T cells were infected with the recombinant retrovirus (retrovirus for expression of anti-GPC3 scFv CAR, etc.), to introduce the retroviral vector for expression of anti-GPC3 scFv CAR, etc. into the T cells. Specifically, peripheral blood mononuclear cells were isolated from the peripheral blood of a healthy person according to a conventional method and then activated and cultured for 48 hours in the presence of immobilized anti-CD3 monoclonal antibody clone (OKT3) (5 µg/mL) and retronectin (R) available from Takara Bio Inc. (25 µg/mL), and IL-2. A virus adsorption plate was produced by adding a culture supernatant containing a recombinant retrovirus for expression of anti-GPC3 scFv CAR, etc. onto a plate coated with retronectin, followed by centrifugation at 4°C and 2000 g for two hours. The human T cells activated and cultured were seeded at 1 × 10⁵ cells/mL onto the virus adsorption plate produced and cultured overnight in the presence of IL-2. On the next day, the T cells were collected and seeded onto a new virus adsorption plate to perform the second viral infection. After culturing for 4 hours, the cells were collected, and 3 times the amount of the culture solution was added thereto. The cells were seeded on a cell culture plate and then cultured for 3 days.

### 2. Method

### [Flow cytometry analysis]

Flow cytometry analysis was performed to confirm that the human T cells infected with the retrovirus for expression of anti-GPC3 scFv CAR, etc. express an anti-GPC3 scFv CAR. Specifically, the human T-cell group (T-cell group containing the anti-GPC3 scFv CAR-T cells) infected with the retrovirus for expression of anti-GPC3 scFv CAR, etc. was incubated on ice for 30 minutes in the presence of a biotinylated recombinant GPC3 protein (available from R&D Systems). A FACS Buffer (1% BSA/PBS solution) was added thereto, followed by centrifugation, to remove the supernatant. Thereafter, an APC-labeled anti-CD8 antibody (available from BioLegend, Inc.) and BV421-labeled streptavidin (available from BioLegend, Inc.) were added thereto, and the mixture was incubated on ice for 30 minutes. A flow cytometer (BD LSR Fortessa) (available from BD Biosciences) was used for detecting APC and BV421 and measuring the fluorescence levels thereof. As a negative control, a human T-cell group (T-cell group) uninfected with the retrovirus for expression of anti-GPC3 scFv CAR, etc. was subjected to flow cytometry analysis in the same manner.

### [Injury activity on GPC3-expressing cancer cells]

In order to confirm the cytotoxic activity of the anti-GPC3 scFv CAR-T cell on cancer cells, GPC3-expressing cancer cells were cultured in the presence of the T-cell group containing the anti-GPC3 scFv CAR-T cells. Specifically, the T-cell group containing the anti-GPC3 scFv CAR-T cells was mixed with a hepatocellular carcinoma-derived cell line (Sk-HEP-1 cells) (purchased from ECACC) or GPC3-expressing Sk-HEP-1 cells (Sk-HEP-1 GPC3 cells) at 1:1 (1 × 10⁵/well), and the mixture was cultured in a 24-well plate. 48 hours thereafter, the cells were collected and incubated on ice for 30 minutes in the presence of a Phycoerythrin-labeled anti-CD45 antibody (available from BioLegend, Inc). A FACS Buffer (1% BSA/PBS solution) was added thereto, followed by centrifugation, to remove the supernatant. Thereafter, a flow cytometer (BD LSR Fortessa) (available from BD Biosciences) was used for detecting Phycoerythrin and analyzing the fluorescence level, and the numbers of CD45-positive cells (corresponding to the T cells) and CD45-negative cells (corresponding to remaining Sk-HEP-1 GPC3 cells) and the ratio thereof were measured.

### [IFN-γ producibility of anti-GPC3 scFv CAR-T cells]

Whether or not the anti-GPC3 scFv CAR-T cells have interferon gamma (IFN-γ) producibility was analyzed. Specifically, the T-cell group containing the anti-GPC3 scFv CAR-T cells ("CAR-T cells" in Figure 3) or a T-cell group without gene introduction ("T cells" in Figure 3) was mixed with Sk-HEP-1 cells or Sk-HEP-1 GPC3 cells at 1:1 (1 × 10⁵/well each), the mixture was cultured in a 24-well plate for 48 hours, and the concentration of IFN-γ produced in the culture supernatant was measured by ELISA (available from BioLegend, Inc).

### [IL-7 and CCL19 producibility of anti-GPC3 scFv CAR-T cells]

Whether or not the anti-GPC3 scFv CAR-T cells have IL-7 and CCL19 producibility was analyzed. Specifically, the T-cell group containing the anti-GPC3 scFv CAR-T cells ("CAR-T cells" in Figure 4) or a T-cell group without gene introduction ("T cells" in Figure 4) was mixed with Sk-HEP-1 cells or Sk-HEP-1 GPC3 cells at 1:1 (1 × 10⁵/well each), the mixture was cultured in a 24-well plate for 48 hours, and the concentrations of IL-7 and CCL19 produced in the culture supernatant were measured by ELISA (available from R&D Systems) . As a negative control, a CAR T-cell group without gene introduction (T cells) with Sk-HEP-1 cells or Sk-HEP-1 GPC3 cells was cultured under the same conditions.

### 3. Results

### [Flow cytometry analysis]

It was shown that 48.1% of cells in the human T-cell group infected with the retrovirus for expression of anti-GPC3 scFv CAR, etc. were CAR positive in which BV421 was detected (see Figure 1B). Meanwhile, almost no CAR-positive cells were observed in the retrovirus-uninfected human T-cell group (see Figure 1A). These results demonstrated that about 50% of cells in the human T-cell group infected with the retrovirus for expression of anti-GPC3 scFv CAR, etc. were T cells expressing the anti-GPC3 scFv CAR (anti-GPC3 scFv CAR-T cells).

### [Injury activity on GPC3-expressing cancer cells]

As compared with the case of using the T-cell group uninfected with the retrovirus for expression of anti-GPC3 scFv CAR, etc., the T-cell group containing the anti-GPC3 scFv CAR-T cells exhibited very high injury activity on Sk-HEP-1 GPC3 cells ("GPC3" in Figure 2) and killed most of the Sk-HEP-1 GPC3 cells (see Figure 2). Meanwhile, the T-cell group containing the anti-GPC3 scFv CAR-T cells exhibited almost no injury activity on Sk-HEP-1 cells ("mock" in Figure 2), like the T-cell group (see Figure 2). These results demonstrated that the anti-GPC3 scFv CAR-T cells exert cytotoxic activity specific for cancer cells expressing GPC3.

### [IFN-γ producibility of anti-GPC3 scFv CAR-T cells]

The T-cell group containing the anti-GPC3 scFv CAR-T cells exhibited very high IFN-γ producibility in the presence of Sk-HEP-1 GPC3 cells, as compared with the case of using the T-cell group uninfected with the retrovirus for expression of anti-GPC3 scFv CAR, etc. (see Figure 3). Meanwhile, the T-cell group containing the anti-GPC3 scFv CAR-T cells exhibited almost no IFN-γ producibility in the presence of Sk-HEP-1 cells, like the T-cell group (see Figure 3). These results demonstrated that the anti-GPC3 scFv in the anti-GPC3 scFv CAR-T cells were bound to GPC3 surface antigens in the cancer cells and activated, as a result of which IFN-γ was produced.

### [IL-7 and CCL19 producibility of anti-GPC3 scFv CAR-T cells]

The T-cell group containing the anti-GPC3 scFv CAR-T cells produced a low value of IL-7 and CCL19 in the presence of Sk-HEP-1 cells but produced a further high level of IL-7 and CCL19 in the presence of Sk-HEP-1 GPC3 cells (see Figure 4). In the T-cell group uninfected with the retrovirus for expression of anti-GPC3 scFv CAR, etc., IL-7 production and CCL19 production were hardly observed in the presence of both Sk-HEP-1 cells and Sk-HEP-1 GPC3 cells. These results demonstrated that the anti-GPC3 scFv CAR-T cells produced IL-7 and CCL19.

### Industrial Applicability

The present invention contributes to the cancer immunotherapy field using immunocompetent cells.

## Claims

1. A chimeric antigen receptor (CAR) comprising:
a single-chain antibody that specifically binds to a human GPC3 (Glypican-3)-derived polypeptide consisting of the amino acid sequence shown by SEQ ID No: 11;
a transmembrane region fused to a carboxyl terminus of the single-chain antibody; and
an immunocompetent cell activation signaling transduction region fused to the carboxyl terminus of the transmembrane region, wherein
the single-chain antibody comprises:
a heavy chain complementarity determining region (CDR) 1 consisting of the amino acid sequence shown by SEQ ID No: 1, a heavy-chain CDR2 consisting of the amino acid sequence shown by SEQ ID No: 2, and a heavy-chain CDR3 consisting of the amino acid sequence shown by SEQ ID No: 3; and
a light-chain CDR1 consisting of the amino acid sequence shown by SEQ ID No: 4, a light-chain CDR2 consisting of the amino acid sequence shown by SEQ ID No: 5, and a light-chain CDR3 consisting of the amino acid sequence shown by SEQ ID No: 6.

2. The CAR according to claim 1, wherein
the single-chain antibody comprises:
a heavy-chain variable region consisting of an amino acid sequence having at least 80% or more sequence identity with the amino acid sequence shown by SEQ ID No: 7; and
a light-chain variable region consisting of an amino acid sequence having at least 80% or more sequence identity with the amino acid sequence shown by SEQ ID No: 8.

3. The CAR according to claim 1 or 2, wherein
the single-chain antibody comprises:
an amino acid sequence having at least 80% or more sequence identity with the amino acid sequence shown by SEQ ID No: 12; or
an amino acid sequence having at least 80% or more sequence identity with the amino acid sequence shown by SEQ ID No: 13.

4. The CAR according to any one of claims 1 to 3, wherein
the transmembrane region and the immunocompetent cell activation signaling transduction region fused to the carboxyl terminus of the transmembrane region comprise an amino acid sequence having at least 80% or more sequence identity with an amino acid sequence shown by any one of SEQ ID Nos: 14 to 16.

5. An immunocompetent cell expressing the CAR according to any one of claims 1 to 4.

6. The immunocompetent cell according to claim 5, further expressing interleukin 7 (IL-7) and chemokine ligand 19 (CCL19).

7. An anticancer agent comprising:
the immunocompetent cell according to claim 5 or 6; and
a pharmaceutically acceptable additive.

8. A CAR gene encoding the CAR according to any one of claims 1 to 4.

9. A vector comprising:
a promoter; and
the CAR gene encoding the CAR according to claim 8 operably linked downstream of the promoter.
